# DEMANDE DE BREVET EUROPEEN

(11) **EP 1 391 952 A2**
(43) Date de publication de la demande: **25.02.2004**
(21) Numéro de dépôt: 03292436.7
(22) Date de dépôt: 30.12.1997
(51) Int. Cl.: H01M 6/16, H01M 10/40

(54) **Sels d'anions pentacycliques ou dérivés de tétrazapentalène, et leurs utilisations comme matériaux à conduction ionique**

(30) Priorité: 30.12.1996 CA 2194127; 05.03.1997 CA 2199231
(62) Demande divisionnaire de: 97403188.2
(71) Demandeur: HYDRO-QUEBEC, Montréal Québec H2Z 1A4 (CA); CENTRE NATIONAL DE LA RECHERCHE SCIENTIFIQUE (CNRS), 75016 Paris (FR)
(72) Inventeur: Armand, Michel, Montréal, Québec H3T (CA); Michot, Christophe, Montréal, Québec (CA); Gauthier, Michel, La Prairie, Québec J5R 1E6 (CA); Choquette, Yves, Sainte-Julie, Québec J3E 1P4 (CA)
(74) Mandataire: Sueur, Yvette

(57) **Abrégé**

L'invention concerne des composés ioniques dans lesquels la charge anionique est délocalisée.

Un composé de l'invention comprend une partie anionique associée à au moins une partie cationique M^{m+} en nombre suffisant pour assurer la neutralité électronique de l'ensemble. M est un hydroxonium, un nitrosonium NO⁺, un ammonium -NH₄⁺, un cation métallique ayant la valence m, un cation organique ayant la valence m ou un cation organométallique ayant la valence m. La charge anionique est portée par un noyau pentacyclique ou dérivé de tétrazapentalène portant des substituants électroattracteurs.

Les composés sont utiles notamment pour les matériaux à conduction ionique, les matériaux à conduction électronique, les colorants et la catalyse de diverses réactions chimiques.

## Description

La présente invention a pour objet des composés ioniques dans lesquels la charge anionique est portée par un noyau pentacyclique ou dérivé de tétrazapentalène, et leurs utilisations.

Les dérivés des anions non nucléophiles ou peu basiques présentent une importance croissante dans toutes les applications de la chimie pour stabiliser ou activer des charges cationiques diverses telles que celles des colorants ou des espèces intermédiaires dans les polymérisations. Ils interviennent également comme intermédiaires pour des réactions variées de la chimie organique. En électrochimie, il est de plus en plus fait appel à des milieux autres que l'eau pour des applications telles que les générateurs primaires ou secondaires, les supercapacités, les systèmes de modulation de la lumière. L'introduction d'une faible conductivité ionique dans les matériaux usuels (polymères, liquides combustibles), permet de dissiper les charges électrostatiques.

On connaît principalement les dérivés des anions de coordination, de type BF₄⁻, PF₆⁻, AsF₆⁻, mais ceux-ci présentent un stabilité limitée du fait de l'équilibre de dissociation libérant l'anion fluorure et l'acide de Lewis correspondant, les deux amenant des réactions parasites et présentant une certaine toxicité. L'anion perchlorate ClO₄⁻ est thermiquement instable et dangereux.

On connaît par ailleurs les anions dérivés des perfluoroalkylsulfonates et surtout bis (perfluoroalkylsulfonyl)imides qui présentent des propriétés intéressantes. Toutefois, la chimie de ces composés est relativement difficile à maîtriser, en particulier la préparation des précurseurs de type R_{F}SO₂―. Il est en outre connu que les hydrocarbures comme le cyclopentadiène forment assez facilement des sels par déprotonation, mais leur acidité et la stabilité de l'anion restent néanmoins très insuffisantes (pKa ≈ 16).

Les inventeurs ont trouvé que, de manière surprenante, les propriétés des composés dérivés du cyclopentadiène étaient considérablement modifiées lorsqu'un carbone du cycle était remplacé par un élément plus électronégatif que le carbone, ou lorsqu'un substituant électronégatif était fixé à un atome de carbone du cycle. Selon le choix des substituants, ces composés donnent des sels facilement solubles et fortement dissociés, y compris dans les milieux organiques moins polaires que l'eau. Ces sels ont des propriétés intéressantes pour de nombreuses applications et leur préparation fait appel à des matériaux plus faciles d'accès. Il est notamment possible d'obtenir des hétérocycles anioniques stables incorporant des quantités plus faibles, voire nulles, de fluor, ou à partir de composés fluorés faciles d'accès.

Il est connu et particulièrement intéressant d'introduire des groupements ioniques dans les molécules ou les polymères organiques possédant des fonctions variées. Les forces coulombiennes correspondent, en effet, aux interactions les plus fortes disponibles au niveau moléculaire, et les groupements ioniques modifient de la manière la plus marquée les molécules auxquelles ils sont attachés. On peut citer les colorants qui sont rendus solubles dans l'eau à l'aide de fonctions sulfonates ou carboxylates. Cependant les groupements de ce type -CO₂⁻ 1/mM^{m+} ou -SO₃⁻ 1/mM^{m+} ne sont pas dissociés, et ils n'induisent pas de solubilité dans les solvants autres que l'eau ou certains solvants protiques très polaires comme les alcools légers, ce qui restreint considérablement la portée de leur utilisation.

Les possibilités de substitution liées à la chimie des composés dérivés des cycles pentagonaux de l'invention permet donc aussi d'introduire des groupements ioniques dans des molécules variées.

La présente invention a par conséquent pour objet une famille de composés ioniques possédant une bonne solubilité et une bonne dissociation, sans qu'il soit nécessaire de faire appel à des modifications complexes de la molécule de départ. Les précurseurs des molécules de l'invention sont en général facilement accessibles.

Un composé ionique de la présente invention comprend au moins une partie anionique associée à au moins une partie cationique M en nombre suffisant pour assurer la neutralité électronique de l'ensemble, caractérisé en ce que M est un hydroxonium, un nitrosonium NO⁺, un ammonium -NH₄⁺, un cation métallique ayant la valence m, un cation organique ayant la valence m ou un cation organométallique ayant la valence m, et en ce que la partie anionique est pentacyclique ou dérivée de tétrazapentalène et répond à l'une des formules suivantes : 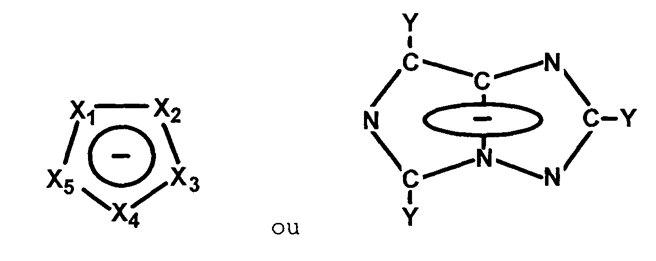 dans lesquelles les groupes -Xᵢ- représentent indépendamment les uns des autres un groupe choisi parmi -N=, -N⁻-, -C(Y_{C})=, -C⁻(Y_{C})-, -S(=O)(Q_{S})=, -S(Q_{S})= ou -P(Q')(Q")=, étant entendu que, parmi les cinq groupes -Xᵢ- formant le cycle, au plus quatre groupes -Xᵢ- comprennent un atome d'azote, au plus deux groupes -Xᵢ- comprennent un atome de soufre à condition qu'ils ne soient pas adjacents sur le cycle, au plus un groupe -Xᵢ- comprend un atome de phosphore, et :
- Q' et Q" représentent indépendamment l'un de l'autre un radical perhaloalkyle ou perhaloalkényle en C₁-C₈, un radical aryle ou alkylaryle en C₆-C₁₂ éventuellement halogéné, chacun pouvant contenir des substituants oxa, thia, aza ;
- Q_{S} est un radical choisi parmi :
   a) les radicaux alkyle ou alkényle, les radicaux aryle, arylalkyle, alkylaryle ou alkénylaryle, les radicaux alicycliques ou hétérocycliques, y compris les radicaux polycycliques, lesdits radicaux étant éventuellement halogénés ou perhalogénés et/ou portant éventuellement au moins un groupe fonctionnel éther, thioéther, amine, imine, amide, carboxyle, carbonyle, isocyanate, isothiocyanate, hydroxy ;
   b) les radicaux aromatiques monocycliques, polycycliques ou condensés dans lesquels les noyaux aromatiques et/ou au moins un substituant d'un noyau comprennent des hétéroatomes tels que l'azote, l'oxygène, le soufre ;
   c) les radicaux polymères,
   d) les radicaux possédant un ou plusieurs groupements ionophores cationiques et/ou un ou plusieurs groupements ionophores anioniques ;
- Y_{C} ou Y représente H, ou un groupement attracteur d'électrons choisi dans le groupe constitué par :
   * F, Cl, Br, -C≡N, -S-C≡N, -N=C=S, -N=C=O, -NO₂, CₙF₂ₙ₊₁-CₙF₂ₙ₊₁-O-, CₙF₂ₙ₊₁-CH₂-, -OC₂F₄H, -SCF₃, -SCₙF₂ₙ₊₁, -SC₂F₄H, -OCF=CF₂, -SCF=CF₂, FSO₂ ;
   * les radicaux QSO₂-, -CO₂Q, Q-N-SO₂-, QCO-, dans lesquels Q est choisi parmi les substituants définis ci-dessus pour Q_{S} ;
   * les radicaux comprenant un ou plusieurs noyaux aromatiques contenant éventuellement au moins un atome d'azote, d'oxygène, de soufre ou de phosphore, lesdits noyaux pouvant être éventuellement des noyaux condensés et/ou lesdits noyaux pouvant éventuellement porter au moins un substituant choisi parmi les halogènes, -CN, -NO₂, -SCN, -N₃, CF₂=CF-O-, les radicaux R_{F}- et R_{F}CH₂- dans lesquels R_{F} est un radical perfluoroalkyle ayant de 1 à 12 atomes de carbone, les groupes fluoroalkyloxy, les groupes fluoroalkylthioxy, les radicaux alkyles, alkényles, oxaalkyles, oxaalkényles, azaalkyles, azaalkényles, thiaalkyles, thiaalkényles, les radicaux polymères, les radicaux possédant au moins un groupement ionophore cationique et/ou au moins un groupement ionophore anionique ;

   - ou bien deux substituants parmi Y_{C}, Q_{S}, Q' et Q" d'une part, ou deux substituants Y d'autre part, forment ensemble un cycle ayant de 4 à 8 chaînons, ledit cycle étant éventuellement de nature conjuguée aromatique ;
   - ou bien l'un parmi les substituants Y, Y_{C} ou Q_{S} est un radical multivalent (y compris un dendrimère) relié à au moins un groupement anionique pentacyclique ou dérivé de tétrazapentalène tel que défini ci-dessus ;
   - ou bien l'un des substituants Y, Y_{C} ou Q_{S} représente une unité récurrente d'un polymère.

Dans un composé de la présente invention, le cation peut être un cation métallique choisi parmi les cations de métaux alcalins, les cations de métaux alcalino-terreux, les cations de métaux de transition, les cations de métaux trivalents, les cations de terres rares. A titre d'exemple, on peut citer Na⁺, Li⁺, K⁺, Sm³⁺, La³⁺, Ho³⁺, Sc³⁺, Al³⁺, Y³⁺, Yb³⁺, Lu³⁺, Eu³⁺.

Le cation peut également être un cation organométallique, notamment un métallocénium. A titre d'exemple, on peut citer les cations dérivés du ferrocène, du titanocène, du zirconocène, d'un indénocénium ou d'un arène métallocénium, les cations des métaux de transition complexés par des ligands de type phosphine possédant éventuellement une chiralité, les cations organométalliques possédant un ou plusieurs groupements alkyles ou aryles fixés d'une manière covalente à un atome ou un groupe d'atome, tels les cations méthylzinc, phénylmercure, trialkylétain ou trialkylplomb. Le cation organo-métallique peut faire partie d'une chaîne polymère.

Selon une variante de l'invention, les composés de l'invention ont un cation organique choisi dans le groupe constitué par les cations R₃O⁺ (oxonium), NR₄⁺ (ammonium), RC(NHR₂)₂⁺ (amidinium), C(NHR₂)₃⁺ (guanidinium), C₅R₆N⁺ (pyridinium), C₃R₅N₂⁺ (imidazolium), C₃R₇N₂⁺ (imidazolinium), C₂R₄N₃⁺ (triazolium), SR₃⁺ (sulfonium), PR₄⁺ (phosphonium), IR₂⁺ (iodonium), (C₆R₅)₃C⁺ (carbonium). Dans un cation donné, les radicaux R peuvent être tous identiques. Mais un cation peut aussi comporter des radicaux R différents les uns des autres. Un radical R peut être un H ou bien il est choisi parmi les radicaux suivants :
- les radicaux alkyles, alkényles, oxaalkyles, oxaalkényles, azaalkyles, azaalkényles, thiaalkyles, thia-alkényles, silaalkyles, silaalkényles, aryles, arylalkyles, alkylaryles, alkényl-aryles, dialkylamino et dialkylazo ;
- les radicaux cycliques ou hétérocycliques comprenant éventuellement au moins une chaîne latérale comprenant des hétéroatomes tels que l'azote, l'oxygène, le soufre ;
- les radicaux cycliques ou hétérocycliques comprenant éventuellement des hétéroatomes dans le noyau aromatique ;
- les groupes comprenant plusieurs noyaux aromatiques ou hétérocycliques, condensés ou non, contenant éventuellement au moins un atome d'azote, d'oxygène, de soufre ou de phosphore.

Lorsqu'un cation onium porte au moins deux radicaux R différents de H, ces radicaux peuvent former ensemble un cycle aromatique ou non, englobant éventuellement le centre portant la charge cationique.

Lorsque la partie cationique d'un composé de l'invention est un cation onium, il peut se présenter soit sous la forme d'un groupe cationique indépendant qui n'est lié à la partie anionique que par la liaison ionique entre la charge positive du cation et la charge négative de la partie anionique pentacyclique ou dérivée de tétrazapentalène. Dans ce cas, la partie cationique peut faire partie d'une unité récurrente d'un polymère.

Un cation onium peut également faire partie d'un substituant Y, Y_{C} ou Q_{S} porté par le centre anionique pentacyclique ou dérivé de tétrazapentalène. Dans ce cas, un composé de l'invention constitue un zwitterion.

Lorsque le cation d'un composé de l'invention est un cation onium, il peut être choisi de telle sorte à introduire dans le composé des substituants permettant de conférer audit composé des propriétés spécifiques. Par exemple, le cation M⁺ peut être un hétérocycle cationique à caractère aromatique, comportant au moins un atome d'azote alkylé dans le cycle. A titre d'exemple, on peut citer un imidazolium, un triazolium, un pyridinium, un 4-diméthylamino-pyridinium, lesdits cations portant éventuellement un substituant sur les atomes de carbone du cycle. Parmi ces cations, ceux qui donnent un composé ionique selon l'invention dont le point de fusion est inférieur à 150°C sont particulièrement préférés. Un tel composé possédant une température de fusion basse est particulièrement utile pour l'élaboration de matériaux à conduction protonique. Un matériau à conduction protonique particulièrement préféré comprend un composé selon l'invention dans lequel le cation est formé par addition d'un proton sur l'azote d'une imidazoline, d'un imidazole ou d'un triazole, ainsi que la base azotée correspondante dans une proportion de 0,5 à 10 en rapport molaire.

Un composé de l'invention dans lequel le cation M est un groupement cationique possédant une liaison -N=N-, -N=N⁺, un groupement sulfonium, un groupement iodonium, ou un cation arène-ferrocénium substitué ou non, éventuellement incorporé dans une trame polymérique, est intéressant dans la mesure où il est activable par une source d'énergie actinique de longueur d'onde appropriée. Comme exemples particuliers de tels composés, on peut citer ceux dans lesquels le cation est un cation diaryliodonium, un cation dialkylaryliodonium, un cation triarylsulfonium, un cation trialkylaryle sulfonium, ou un cation phénacyl-dialkyl sulfonium substitué ou non. Les cations précités peuvent faire partie d'une chaîne polymère.

Le cation M d'un composé de l'invention peut incorporer un groupement 2,2'[Azobis(2-2'-imidazolinio-2-yl)propane]²⁺ ou 2,2'-Azobis(2-amidiniopropane)²⁺. Le composé de l'invention est alors capable de libérer, sous l'action de la chaleur ou d'un rayonnement ionisant, des radicaux qui permettent d'initier des réactions de polymérisation, de réticulation ou, d'une manière générale, des réactions chimiques mettant en jeu des radicaux libres. De plus, ces composés sont aisément solubles dans les solvants organiques polymères et monomères même de faible polarité, contrairement aux dérivés des anions de type Cl⁻ habituellement associés à ce type de composés. Ils présentent par ailleurs une pression de vapeur négligeable contrairement aux autres amorceurs radicalaires de type peroxyde ou azo, ce qui est un avantage considérable pour la mise en oeuvre de polymères en films minces, la volatilité de l'amorceur ayant pour conséquence une mauvaise polymérisation ou réticulation de la surface du film.

Dans un mode réalisation de l'invention, l'un au moins des substituants Q_{S} ou Q est choisi parmi les radicaux alkyle, alkényle, oxaalkyle, oxaalkényle, azaalkyle, azaalkényle, thiaalkyle ou thiaalkényle ayant de 1 à 24 atomes de carbone, ou parmi les radicaux aryle, arylalkyle, alkylaryle ou alkénylaryle ayant de 5 à 24 atomes de carbones, ou parmi les radicaux alkyles ou alkényles ayant de 1 à 12 atomes de carbone et comprenant éventuellement au moins un hétéroatome O, N ou S dans la chaîne principale ou dans une chaîne latérale, et/ou portant éventuellement un groupe hydroxy, un groupe carbonyle, un groupe amine, un groupe carboxyle. Q_{S} ou Q peuvent également faire partie d'un radical poly(oxyalkylène) ou d'un radical polystyrène.

Une autre catégorie de composés selon l'invention comprend les composés dans lesquels l'un des substituants Y, Y_{C} ou Q_{S} possède au moins un groupement ionophore anionique et/ou au moins un groupement ionophore cationique. Le groupement anionique peut par exemple être une fonction carboxylate (-CO₂⁻), une fonction sulfonate (-SO₃⁻), une fonction sulfonimide (-SO₂NSO₂-) ou une fonction sulfonamide (-SO₂N-). Le groupement ionophore cationique peut par exemple être un groupement iodonium, sulfonium, oxonium, ammonium, amidinium, guanidinium, pyridinium, imidazolium, imidazolinium, triazolium, phosphonium ou carbonium. Le groupement ionophore cationique peut jouer totalement ou partiellement le rôle du cation M.

Lorsque l'un au moins des substituants Y, Y_{C} ou Q_{S} comporte au moins une insaturation éthylénique et/ou un groupe condensable et/ou un groupe dissociable par voie thermique, par voie photochimique ou par dissociation ionique, les composés de l'invention sont des composés réactifs qui peuvent être soumis à des polymérisations, des réticulations ou des condensations, éventuellement avec d'autres monomères. Ils peuvent également être utilisés pour fixer des groupes ionophores sur les polymères portant la fonction réactive appropriée.

Dans un composé de l'invention, l'un au moins des substituants Y, Y_{C} ou Q_{S} peut être un groupe mésomorphe ou un groupe chromophore ou un polymère conducteur électronique autodopé ou un alcoxysilane hydrolysable.

Un substituant Y, Y_{C} ou Q_{S} peut comporter un groupement susceptible de piéger les radicaux libres, par exemple un phénol encombré ou une quinone.

Un substituant Y, Y_{C} ou Q_{S} peut aussi comporter un dipôle dissociant, par exemple une fonction amide, une fonction sulfonamide ou une fonction nitrile

Un substituant Y, Y_{C} ou Q_{S} peut aussi comporter un couple rédox, par exemple un groupe disulfure, un groupe thioamide, un groupe ferrocène, un groupe phénothiazine, un groupe bis(dialkylaminoaryle), un groupe nitroxyde ou un groupe imide aromatique.

Un substituant Y, Y_{C} ou Q_{S} peut également comprendre un groupe optiquement actif ou un ligand complexant.

Une autre catégorie de composés comprend les composés dans lesquels Y ou Y_{C} représente un acide aminé, ou un polypeptide optiquement ou biologiquement actif.

Dans un mode de réalistion particulier, les substituants Y ou Y_{C} d'un composé de l'invention sont différents d'un groupe perfluoroalkylsulfonyle lorsque M est un cation métallique.

Dans un composé de l'invention, l'un des substituants Y, Y_{C} ou Q_{S} peut être un radical ayant une valence v supérieure à deux, comportant à chacune de ses extrémités libres un groupe pentacyclique anionique. De préférence, ledit radical multivalent comprenant au moins un groupe -SO₂-, un groupe - CO-, un groupe perfluoroalkylène ayant de 2 à 8 atomes de carbone, un groupe phénylène éventuellement substitué par des hétéroatomes, un groupement -(W=W)ₙ- ou un groupement cationique -(W=W)ₙW⁺-, dans lesquels W est un atome d'azote ou un groupement CR dans lequel R représente un radical organique et 0≤n≤5, R étant un atome d'hydrogène ou un radical alkyle ayant de 1 à 8 atomes de carbone, ou deux substituants R formant ensemble un cycle. Dans ce cas, les charges négatives présentes sur la partie anionique pentacyclique ou dérivée de tétrazapentalène du composé de l'invention devront être compensées par le nombre approprié de cations ou de groupes ionophores cationiques M.

L'un des substituants Y, Y_{C} ou Q_{S} peut également représenter une unité récurrente d'une chaîne polymère. Le composé de l'invention se présente alors sous la forme d'un polymère dans lequel au moins une partie des unités récurrentes portent un groupe latéral sur lequel est fixé un groupe anionique pentacyclique ou dérivé de tétrazapentalène.

Dans un mode de réalisation de l'invention, Y ou Y_{C} est avantageusement choisi dans le groupe constitué par -CN, -OCₙF₂ₙ₊₁, -OC₂F₄H, -SCₙF₂ₙ₊₁ et -SC₂F₄H, -O-CF=CF₂, -SCF=CF₂, n étant un nombre entier de 1 à 8. Y ou Y_{C} peut également être un radical CₙF₂ₙ₊₁CH₂-, n étant un nombre entier de 1 à 8, ou parmi les hétérocycles, en particulier ceux dérivés de la pyridine, de la pyrazine, de la pyrimidine, de l'oxadiazole, du thiadiazole, fluorés ou non.

Les composés ioniques de la présente invention comprennent au moins un groupement ionophore sur lequel sont fixés des substituants qui peuvent être très variés. Compte tenu du grand choix possible pour les substituants, les composés de l'invention permettent d'induire des propriétés de conduction ionique dans la plupart des milieux organiques, liquides ou polymères possédant une polarité, même faible. Les applications sont importantes dans le domaine de l'électrochimie, en particulier du stockage de l'énergie dans des générateurs primaires ou secondaires, dans les supercapacités, dans les piles à combustibles et dans les diodes électroluminescentes. La compatibilité des composés ioniques de l'invention avec les polymères ou les liquides organiques permet d'induire des propriétés antistatiques marquées, même lorsque la teneur en composé ionique est extrêmement faible. Les composés de l'invention qui sont des polymères, de même que des composés polymères obtenus à partir de composés de l'invention ayant la propriété de se polymériser ou de se copolymériser, présentent les propriétés énumérées ci-dessus avec l'avantage d'avoir une charge anionique immobile. C'est pourquoi un autre objet de la présente invention est constitué par un matériau à conduction ionique constitué par un composé ionique de la présente invention en solution dans un solvant.

Dans un mode de réalisation, le composé ionique utilisé pour l'élaboration d'un matériau à conduction ionique est choisi parmi les composés dont le cation est l'ammonium, ou un cation dérivé d'un métal, en particulier le lithium ou le potassium, le zinc, le calcium, les métaux des terres rares, ou un cation organique, tel qu'un ammonium substitué, un imidazolium, un triazolium, un pyridinium, un 4-diméthylamino-pyridinium, lesdits cations portant éventuellement un substituant sur les atomes de carbone du cycle. Le matériau à conduction ionique ainsi obtenu présente une conductivité et une solubilité dans les solvants élevées, du fait des interactions faibles entre la charge positive et la charge négative. Son domaine de stabilité électrochimique est étendu, et il est stable dans des milieux aussi bien réducteurs qu'oxydants. De plus, les composés qui ont un cation organique et un point de fusion inférieur à 150°C, en particulier les composés de imidazolium, de triazolium, de pyridinium, de 4-diméthylamino-pyridinium présentent une conductivité élevée intrinsèque, même en l'absence de solvant, lorsqu'ils sont en phase fondue.

Les propriétés du matériau à conduction ionique peuvent également être adaptées par le choix des substituants Y, Y_{C} ou Q d'une part, Q_{S} d'autre part.

Le choix pour Q ou Q_{S} d'un groupe alkyle, d'un groupe aryle, d'un groupe alkylaryle ou d'un groupe arylalkyle, permet d'induire dans le matériau à conduction ionique des propriétés de type mésogène, en particulier les groupements alkyles de 6 à 20 atomes de carbones, les groupements arylealkyle, en particulier ceux contenant l'entité biphényle qui forment des phases de type cristal liquide. Des propriétés de conduction dans des phases de type cristal liquide, nématique, cholestérique ou discotique, sont intéressantes pour les applications relatives à l'affichages optique ou pour réduire la mobilité des anions dans les électrolytes, en particulier dans les électrolytes polymères, sans affecter la mobilité des cations. Cette particularité est importante pour les applications dans les générateurs électrochimiques, en particulier ceux mettant en jeu les cations lithium.

Lorsque Q ou Q_{S} est un groupe mésomorphe ou un groupe comprenant au moins une insaturation éthylénique et/ou un groupe condensable et/ou un groupe dissociable par voie thermique, par voie photochimique ou par dissociation ionique, le matériau à conduction ionique forme aisément des polymères ou copolymères qui sont des polyélectrolytes, soit intrinsèques quand le polymère porte des groupements solvatants, soit par addition d'un solvant polaire de type liquide ou polymère, ou par mélange avec un tel solvant. Ces produits ont une conductivité uniquement due au cations, ce qui constitue une propriété très utile dans les applications de type générateur électrochimique. En faible fraction molaire dans un copolymère, ils induisent des propriétés antistatiques stables et peu dépendantes de l'humidité et favorisent la fixation de colorants cationiques, cette propriété étant utile pour les fibres textiles et les lasers à colorants.

La présence d'un substituant Q ou Q_{S} qui est un polymère conducteur électronique autodopé, améliore la stabilité du matériau à conduction ionique par rapport aux agents extérieurs. La conductivité est stable dans le temps même à des températures élevées. En contact avec les métaux, ces matériaux donnent des résistances d'interface très faibles et protègent en particulier les métaux ferreux ou l'aluminium de la corrosion.

Lorsqu'un substituant Q ou Q_{S} est un alcoxysilane hydrolysable, le matériau à conduction ionique peut former des polymères stables par simple mécanisme d'hydrolyse-condensation en présence d'eau, permettant ainsi de traiter les surfaces d'oxydes, de silice, de silicates, en particulier le verre, pour induire des propriétés de conduction de surface, des propriétés antistatiques, ou pour favoriser l'adhésion de polymères polaires.

Lorsqu'un substituant Y, Y_{C} ou Q_{S} est un groupe comprenant un piège à radicaux libres tel qu'un phénol encombré, ou une quinone, le matériau à conduction ionique présente les avantages et propriétés suivantes : il agit comme antioxydant ne présentant pas de volatilité et compatible avec les monomères et polymères polaires, auquel il confère de surcroît des propriétés antistatiques.

Lorsqu'un substituant Y, Y_{C} ou Q_{S} comprend un dipôle dissociant tel qu'une amide, une sulfonamide ou un nitrile, le matériau à conduction ionique a une conductivité améliorée dans des milieux de faible et moyenne polarité, en particulier dans les polymères solvatants, ce qui permet de minimiser, voire de supprimer l'addition de solvants ou de plastifiants volatils.

La présence d'un substituant Y, Y_{C} ou Q_{S} qui contient un couple rédox tel qu'un disulfure, une thioamide, un ferrocène, une phéno-thiazine, un groupe bis(dialkylaminoaryle), un nitroxyde, un imide aromatique, permet d'induire dans le matériau à conduction ionique des propriétés de navette rédox utiles comme élément de protection et d'égalisation de charge des générateurs électrochimiques, dans les systèmes photoélectrochimiques, en particulier de conversion de la lumière en électricité, dans les systèmes de modulation de la lumière de type électrochrome.

La présence d'un substituant Y, Y_{C} ou Q_{S} qui est un ligand complexant dans un matériau à conduction ionique permet de chélater les cations métalliques, en particulier ceux qui possèdent un charge élevée (2, 3 et 4), sous forme de complexe soluble dans les milieux organiques, y compris dans les milieux aprotiques, et permet le transport de ces cations en particulier sous forme de complexe anionique, dans les polymères solvatants. Les cations métalliques de charge élevée sont en effet immobiles dans les polymères solvatants. Ce type de complexant donne avec certains cations des métaux de transition (Fe, Co...) ou de certaines terres rares (Ce, Eu...) des couples rédox particulièrement stables.

Les matériaux à conduction ionique contenant un composé de l'invention dans lequel un substituant Q ou Q_{S} est un substituant alkyle ou alkényle qui contient au moins un hétéroatome choisi parmi O, N et S ont un pouvoir complexant, et plastifiant, en particulier dans les polymères polaires et tout spécialement les polyéthers. Les hétéroatomes N et S sont sélectivement complexants pour les cations des métaux de transition, Zn et Pb.

Lorsqu'un substituant Q ou Q_{S} alkyle ou alkényle porte en outre un groupe hydroxy, un groupe carbonyle, un groupe amine, un groupe carboxyle, un groupe isocyanate ou un groupe thioisocyanate, le composé ionique de l'invention peut donner par polycondensation un polymère ou un copolymère et le matériau à conduction ionique qui contient un tel polymère ou copolymère présente des propriétés de polyélectrolyte.

La présence, dans le matériau à conduction ionique de l'invention, d'un composé dans lequel un substituant Q ou Q_{S} est choisi parmi les radicaux aryle, arylalkyle, alkylaryle ou alkénylaryle, dans lesquels les chaînes latérales et/ou les noyaux aromatiques comprennent des hétéroatomes tels que l'azote, l'oxygène, le soufre, améliore la dissociation et augmente la possibilité de former des complexes suivant la position de l'hétéroatome (pyridine) ou de donner par oxydation duplicative des polymères ou copolymères conjugués (pyrrole, thiophène).

Lorsque le matériau à conduction ionique contient un composé de l'invention dans lequel un substituant Y, Y_{C} ou Q_{S} représente une unité récurrente d'une chaîne polymère, le matériau constitue un polyélectrolyte.

Un composé de l'invention dans lequel le substituant Y ou Y_{C} est choisi dans le groupe constitué par -OCₙF₂ₙ₊₁, -OC₂F₄H, -SCₙF₂ₙ₊₁ et -SC₂F₄H, -OCF=CF₂, -SCF=CF₂, n étant un nombre entier de 1 à 8, est un précurseur de monomères et polymères stables, en particulier vis-à-vis de l'oxygène même à des températures supérieures à 80°C lorsqu'il s'agit des polymères. Un matériau à conduction ionique qui contient un tel composé est donc particulièrement approprié comme électrolyte d'une pile à combustible.

Un matériau à conduction ionique de la présente invention comprend un composé ionique de l'invention en solution dans un solvant.

Le solvant peut être un solvant liquide aprotique, un polymère polaire ou un de leurs mélanges.

Le solvant liquide aprotique est choisi par exemple parmi les éthers linéaires et les éthers cycliques, les esters, les nitriles, les dérivés nitrés, les amides, les sulfones, les sulfolanes, les alkylsulfamides et les hydrocarbures partiellement halogénés. Les solvants particulièrement préférés sont le diéthyléther, le diméthoxyéthane, le glyme, le tétrahydrofurane, le dioxane, le diméthyltétrahydrofurane, le formiate de méthyle ou d'éthyle, le carbonate de propylène ou d'éthylène, les carbonates d'alkyles (notamment le carbonate de diméthyle, le carbonate de diéthyle et le carbonate de méthylpropyle), les butyrolactones, l'acétonitrile, le benzonitrile, le nitrométhane, le nitrobenzène, la diméthylformamide, la diéthylformamide, la N-méthylpyrrolidone, la diméthylsulfone, la tétraméthylène sulfone et les tétraalkylsulfonamides ayant de 5 à 10 atomes de carbone.

Le polymère polaire peut être choisi parmi les polymères solvatants, réticulés ou non, portant ou non des groupes ioniques greffés. Un polymère solvatant est un polymère qui comporte des unités solvatantes contenant au moins un hétéroatome choisi parmi le soufre, l'oxygène, l'azote et le fluor. A titre d'exemple de polymères solvatants, on peut citer les polyéthers de structure linéaire, peigne ou à blocs, formant ou non un réseau, à base de poly(oxyde d'éthylène), ou les copolymères contenant le motif oxyde d'éthylène ou oxyde de propylène ou allylglycidyléther, les polyphosphazènes, les réseaux réticulés à base de polyéthylène glycol réticulé par des isocyanates ou les réseaux obtenus par polycondensation et portant des groupements qui permettent l'incorporation de groupements réticulables. On peut également citer les copolymères à blocs dans lesquels certains blocs portent des fonctions qui ont des propriétés rédox. Bien entendu, la liste ci-dessus n'est pas limitative, et tous les polymères présentant des propriétés solvatantes peuvent être utilisés.

Un matériau à conduction ionique de la présente invention peut comprendre simultanément un solvant liquide aprotique choisi parmi les solvants liquides aprotiques cités ci-dessus et un solvant polymère polaire comprenant des unités contenant au moins un hétéroatome choisi parmi le soufre, l'azote, l'oxygène et le fluor. Il peut comprendre de 2 à 98% de solvant liquide. A titre d'exemple d'un tel polymère polaire, on peut citer les polymères qui contiennent principalement de unités dérivées de l'acrylonitrile, du fluorure de vinylidène, de la N-vinylpyrrolidone ou du méthacrylate de méthyle. La proportion de liquide aprotique dans le solvant peut varier de 2% (correspondant à un solvant plastifié) à 98% (correspondant à un solvant gélifié).

Un matériau à conduction ionique de la présente invention peut contenir en outre un sel utilisé classiquement dans l'art antérieur pour l'élaboration d'un matériau à conduction ionique. Parmi les sels utilisables en mélange avec un composé ionique selon l'invention, on préfère tout particulièrement un sel choisi parmi les perfluoroalcanesulfonates, les bis(perfluoroalkylsulfonyl)imidures, les bis(perfluoroalkylsulfonyl)méthanes et les tris(perfluoroalkylsulfonyl)-méthanes.

Bien entendu, un matériau à conduction ionique de l'invention peut contenir en outre les additifs utilisés de manière classique dans ce type de matériau, et notamment des charges minérales ou organiques sous forme de poudre ou de fibres.

Un matériau à conduction ionique de l'invention peut être utilisé comme électrolyte dans un générateur électrochimique. La présente invention a ainsi pour autre objet un générateur électrochimique comprenant une électrode négative et une électrode positive séparées par un électrolyte, caractérisé en ce que l'électrolyte est un matériau à conduction ionique tel que défini ci-dessus. Selon un mode de réalisation particulier, un tel générateur comprend une électrode négative constituée par du lithium métallique, ou par l'un de ses alliages, éventuellement sous forme de dispersion nanométrique dans de l'oxyde de lithium, ou par un nitrure double de lithium et d'un métal de transition, ou par un oxyde à bas potentiel ayant pour formule générale Li_{1+y+x/3}Ti₂₋ _{x/3}O₄ (0 ≤ x ≤ 1, 0 ≤ y ≤ 1), ou par le carbone et les produit carbonés issus de la pyrolyse de matières organiques. Selon un autre mode de réalisation, le générateur comprend une électrode positive choisie parmi les oxydes de vanadium VOₓ (2 ≤ x ≤ 2,5), LiV₃O₈, Li_{y}Ni₁₋ₓCoₓO₂, (0 ≤ x ≤ 1 ; 0 ≤ y ≤ 1), les spinelles de manganèse Li_{y}Mn₁₋ₓMₓO₂ (M = Cr, Al, V, Ni, 0 ≤ x ≤ 0,5 ; 0 ≤ y ≤ 2), les polydisulfures organiques, FeS, FeS₂, le sulfate de fer Fe₂(SO₄)₃, les phosphates et phosphosilicates de fer et de lithium de structure olivine, ou leurs produits de substitution du fer par le manganèse, utilisés seuls ou en mélanges. Le collecteur de l'électrode positive est de préférence en aluminium.

Un matériau à conduction ionique de la présente invention peut également être utilisé dans une supercapacité. Un autre objet de la présente invention est par conséquent une supercapacité utilisant au moins une électrode de carbone à haute surface spécifique, ou une électrode contenant un polymère rédox, dans laquelle l'électrolyte est un matériau à conduction ionique tel que défini ci-dessus.

Un matériau à conduction ionique de la présente invention peut également être utilisé pour le dopage p ou n d'un polymère à conduction électronique et cette utilisation constitue un autre objet de la présente invention.

En outre, un matériau à conduction ionique de la présente invention peut être utilisé comme électrolyte dans un dispositif électrochrome. Un dispositif électrochrome dans lequel l'électrolyte est un matériau à conduction ionique selon l'invention est un autre objet de la présente invention.

Il a été observé que la forte dissociation des espèces ioniques des composés de l'invention se traduisait par une stabilisation des carbocations, en particulier ceux dans lesquels il existe une conjugaison avec l'oxygène ou l'azote et, d'une manière surprenante, par une forte activité de la forme protonée des composés de l'invention sur certains monomères. La présente invention a donc également pour objet l'utilisation des composés ioniques comme photoinitiateurs sources d'acides de Brønsted catalyseurs de polymérisation ou de réticulation de monomères ou de prépolymères capables de réagir par voie cationique, ou comme catalyseurs pour la modification de polymères.

Le procédé de polymérisation ou de réticulation de monomères ou de prépolymères capables de réagir par voie cationique est caractérisé en ce que l'on utilise un composé de l'invention comme photoinitiateur source d'acide catalysant la réaction de polymérisation. Les composés selon l'invention dans lesquels le cation est un groupement possédant une liaison -N=N⁺, -N=N-, un groupement sulfonium, un groupement iodonium, ou un cation arène-ferrocénium substitué ou non, éventuellement incorporé dans une trame polymérique, sont particulièrement préférés.

Le choix des substituants Y, Y_{C} ou Q_{S} est effectué de manière à augmenter la solubilité dudit composé dans les solvants utilisés pour la réaction des monomères ou des prépolymères, et en fonction des propriétés souhaitées pour le polymère final. Par exemple, le choix de radicaux alkyles non substitués donne une solubilité dans les milieux peu polaires. Le choix de radicaux comprenant un groupe oxa ou une sulfone donnera une solubilité dans les milieux polaires. Les radicaux incluant un groupement sulfoxyde, un groupement sulfone, un groupement oxyde de phosphine, un groupement phosphonate, obtenus respectivement par addition d'oxygène sur les atomes de soufre ou de phosphore, peuvent conférer au polymère obtenu des propriétés améliorées en ce qui concerne l'adhésion, la brillance, la résistance à l'oxydation ou aux UV. Les monomères et les prépolymères qui peuvent être polymérisés ou réticulés à l'aide des photoinitiateurs de la présente invention sont ceux qui peuvent subir une polymérisation cationique.

Parmi les monomères, on peut citer les monomères qui comportent une fonction éther cyclique, une fonction thioéther cyclique ou une fonction amine cyclique les composés vinyliques, (plus particulièrement les éthers vinyliques), les oxazolines, les lactones et les lactames.

Parmi les monomères du type éther ou thioéther cyclique, on peut citer l'oxyde d'éthylène, l'oxyde de propylène, l'oxétane, l'épichlorhydrine, le tétrahydrofurane, l'oxyde de styrène, l'oxyde de cyclohexène, l'oxyde de vinylcyclohexène, le glycidol, l'oxyde de butylène, l'oxyde d'octylène, les éthers et les esters de glycidyle (par exemple le méthacrylate ou l'acrylate de glycidyle, le phényl glycidyl éther, le diglycidyléther de bisphénol A ou ses dérivés fluorés), les acétals cycliques ayant de 4 à 15 atomes de carbone (par exemple le dioxolane, le 1,3-dioxane, le 1,3-dioxépane) et les spiro-bicyclo dioxolanes.

Parmi les composés vinyliques, les éthers vinyliques constituent une famille très importante de monomères sensibles en polymérisation cationique. A titre d'exemple, on peut citer l'éthyl vinyl éther, le propyl vinyl éther, l'isobutyl vinyl éther, l'octadécyl vinyl éther, l'éthylèneglycol monovinyl éther, le diéthylèneglycol divinyl éther, le butanediol monovinyl éther, le butanediol divinyl éther, l'hexanediol divinyl éther, l'éthylèneglycol butyl vinyl éther, le triéthylèneglycol méthyl vinyl éther, le cyclohexanediméthano monovinyl éther, le cyclohexanediméthanol divinyl éther, le 2-éthylhexyl vinyl éther, le poly-THF-divinyl éther ayant une masse comprise entre 150 et 5000, le diéthylèneglycol monovinyl éther, le triméthylolpropane trivinyl éther, l'aminopropyl vinyl éther, le 2-diéthylaminoéthyl vinyl éther.

Comme autres composés vinyliques, on peut citer à titre d'exemple les 1,1-dialkyléthylènes (par exemple l'isobutène), les monomères aromatiques vinyliques (par exemple le styrène, les α-alkylstyrènes, notamment l'α-méthylstyrène, le 4-vinylanisole, l'acénaphtène), les composés N-vinyliques (par exemple la N-vinylpyrolidone ou les N-vinyl sulfonamides).

Parmi les prépolymères, on peut citer les composés dans lesquels des groupements époxy sont portés par une chaîne aliphatique, une chaîne aromatique, ou une chaîne hétérocyclique, par exemple les éthers glycidiques du bisphénol A éthoxylés par 3 à 15 unités d'oxyde d'éthylène, les siloxanes possédant des groupements latéraux du type époxycyclohexèneéthyle obtenus par hydrosilylation des copolymères de dialkyl, d'alkylaryl ou de diaryl siloxane avec le méthyl hydrogénosiloxane en présence d'oxyde de vinylcyclohexène, les produits de condensation du type sol-gel obtenus à partir du triéthoxy ou du triméthoxy silapropylcyclohexène oxyde, les uréthanes incorporant les produits de réaction du butanediol monovinyléther et d'un alcool de fonctionnalité supérieure ou égale à 2 sur un di ou un tri isocyanate aliphatique ou aromatique.

Le procédé de polymérisation selon l'invention consiste à mélanger au moins un monomère ou prépolymère capable de polymériser par voie cationique et au moins un composé ionique de l'invention, et à soumettre le mélange obtenu à un rayonnement actinique ou un rayonnement β. De préférence, le mélange réactionnel est soumis au rayonnement après avoir été mis sous forme d'une couche mince ayant une épaisseur inférieure à 5 mm, de préférence sous forme d'un film mince ayant une épaisseur inférieure ou égale à 500 µm. La durée de la réaction dépend de l'épaisseur de l'échantillon et de la puissance de la source à la longueur d'onde λ active. Elle est définie par la vitesse de défilement devant la source, qui est comprise entre 300 m/min et 1 cm/min. Des couches de matériau final ayant une épaisseur supérieure à 5 mm peuvent être obtenues en répétant plusieurs fois l'opération consistant à épandre une couche et à la traiter par le rayonnement.

Généralement, la quantité de photoinitiateur utilisé est comprise entre 0,01 et 15 % en poids par rapport au poids de monomère ou de prépolymère, de préférence entre 0,1 et 5 % en poids.

Un composé ionique de la présente invention peut être utilisé comme photoinitiateur en l'absence de solvant, notamment lorsque l'on souhaite polymériser des monomères liquides dans lesquels le composé ionique utilisé comme photoinitiateur est soluble ou aisément dispersable. Cette forme d'utilisation est particulièrement intéressante, car elle permet de supprimer les problèmes liés aux solvants (toxicité, inflammabilité).

Un composé ionique de la présente invention peut également être utilisé en tant que photoinitiateur sous forme d'une solution homogène dans un solvant inerte vis-à-vis de la polymérisation, prête à l'emploi et aisément dispersable, en particulier dans le cas où le milieu à polymériser ou à réticuler présente une viscosité élevée.

Comme exemple de solvant inerte, on peut citer les solvants volatils, tels que l'acétone, la méthyl-éthyl cétone et l'acétonitrile. Ces solvants serviront simplement à diluer les produits à polymériser ou à réticuler (pour les rendre moins visqueux, surtout lorsqu'il s'agit d'un prépolymère). Ils seront éliminés après la polymérisation ou la réticulation par séchage. On peut également citer les solvants non volatils. Un solvant non volatil sert également à diluer les produits que l'on veut polymériser ou réticuler, et à dissoudre le composé de l'invention utilisé comme photoinitiateur, mais il restera dans le matériau formé et il agit ainsi comme plastifiant. A titre d'exemple, on peut citer le carbonate de propylène, la γ-butyrolactone, les éther-esters des mono-, di-, tri- éthylène ou propylène glycols, les éther-alcools des mono-, di-, tri- éthylène ou propylène glycols, les plastifiants tels que les esters de l'acide phtalique ou de l'acide citrique.

Dans un autre mode de mise en oeuvre de l'invention, on utilise comme solvant ou diluant un composé réactif vis-à-vis de la polymérisation, qui est un composé de faible masse moléculaire et de faible viscosité qui va jouer à la fois le rôle de monomère polymérisable et le rôle de solvant ou de diluant pour des monomères plus visqueux ou des prépolymères utilisés conjointement. Après la réaction, ces monomères ayant servi de solvant font partie du réseau macromoléculaire finalement obtenu, leur intégration étant plus grande lorsqu'il s'agit de monomères bi-fonctionnels. Le matériau obtenu après irradiation ne contient plus de produits ayant un faible poids moléculaire et une tension de vapeur appréciable, ou susceptibles de contaminer les objets avec lesquels le polymère est en contact. A titre d'exemple, un solvant réactif peut être choisi parmi les mono- et diéthers vinyliques des mono-, di-, tri-, tétraéthylène et propylène glycols, la N-méthylpyrolidone, le 2-propényléther du carbonate de propylène commercialisé par exemple sous la dénomination PEPC par la société ISP, New Jersey, Etats-Unis.

Pour irradier le mélange réactionnel, le rayonnement peut être choisi parmi le rayonnement ultraviolet, le rayonnement visible, les rayons X, les rayons γ et le rayonnement β. Lorsque l'on utilise la lumière ultraviolette comme rayonnement actinique, il peut être avantageux d'ajouter aux photoinitiateurs de l'invention des photosensibilisateurs destinés à permettre une photolyse efficace avec les longueurs d'ondes moins énergétiques que celles correspondant au maximum d'absorption du photoinitiateur, telles que celles émises par les dispositifs industriels, (λ ≈ 300 nm pour les lampes à vapeur de mercure en particulier). De tels additifs sont connus, et à titre d'exemples non limitatifs, on peut citer l'anthracène, le diphényl-9,10-anthracène, le pérylène, la phénothiazine, le tétracène, la xanthone, la thioxanthone, l'acétophénone, la benzophénone, les 1,3,5-triaryl-2-pyrazolines et leurs dérivés, en particulier les dérivés de substitution sur les noyaux aromatiques par des radicaux alkyles, oxa- ou aza-alkyles permettant entre autre de changer la longueur d'onde d'absorption. L'isopropylthioxantone est un exemple de photosensibilisateur préféré lorsque l'on utilise un sel d'iodonium selon l'invention comme photoinitiateur.

Parmi les différents types de rayonnement mentionnés, le rayonnement ultraviolet est particulièrement préféré. D'une part, il est plus commode d'emploi que les autres rayonnements mentionnés. D'autre part, les photoinitiateurs sont en général directement sensibles aux rayons UV et les photosensibilisateurs sont d'autant plus efficaces que la différence d'énergie (δλ) est plus faible.

Les composés ioniques de l'invention peuvent aussi être mis en oeuvre en association avec des amorceurs de type radicalaire générés thermiquement ou par action d'une radiation actinique. Il est ainsi possible de polymériser ou de réticuler des mélanges de monomères ou de prépolymères contenant des fonctions dont les modes de polymérisation sont différents, par exemple des monomères ou des prépolymères polymérisant par voie radicalaire et des monomères ou des prépolymères polymérisant par voie cationique. Cette possibilité est particulièrement avantageuse pour créer des réseaux interpénétrés ayant des propriétés physiques différentes de celles qui seraient obtenues par simple mélange des polymères issus des monomères correspondants. Les éthers vinyliques ne sont pas ou sont peu actifs par amorçage radicalaire. Il est donc possible, dans un mélange réactionnel contenant un photoinitiateur selon l'invention, un amorceur radicalaire, au moins un monomère du type éther vinylique et au moins un monomère comprenant des doubles liaisons non activées telles que celles des groupes allyliques, d'effectuer une polymérisation séparée de chaque type de monomère. Il est par contre connu que les monomères déficients en électrons, tels que les esters ou les amides de l'acide fumarique, de l'acide maléique, de l'acide acrylique ou méthacrylique, de l'acide itaconique, de l'acrylonitrile, du méthacrylonitrile, la maléimide et ses dérivés, forment en présence d'éthers vinyliques riches en électrons, des complexes de transfert de charge donnant des polymères alternés 1:1 par amorçage radicalaire. Un excès initial de monomères vinyliques par rapport à cette stoechiométrie permet de préserver des fonctions polymérisables par initiation cationique pure. Le déclenchement de l'activité d'un mélange d'amorceur radicalaire et d'amorceur cationique selon l'invention peut être fait simultanément pour les deux réactifs dans le cas par exemple d'insolation par un rayonnement actinique d'une longueur d'onde pour laquelle les photoinitiateurs de l'invention et les amorceurs radicalaires choisis sont actifs, par exemple à λ = 250 nm. A titre d'exemple, on peut citer comme amorceurs les produits commerciaux suivants : Irgacure 184®, Irgacure 651®, Irgacure 261®, Quantacure DMB®, Quantacure ITX®.

Il peut aussi être avantageux d'utiliser les deux modes de polymérisation d'une manière séquentielle, pour former dans un premier temps des prépolymères dont la mise en forme est aisée et dont le durcissement, l'adhésion, la solubilité ainsi que le degré de réticulation peuvent être modifiés par le déclenchement de l'activité de l'amorceur cationique. Par exemple, un mélange d'un amorceur radicalaire thermodissociable et d'un photoinitiateur cationique selon l'invention permet de réaliser des polymérisations ou des réticulations séquentielles, d'abord sous l'action de la chaleur, puis sous l'action d'un rayonnement actinique. D'une manière similaire, si l'on choisit un amorceur radicalaire et un photoinitiateur cationique selon l'invention, le premier étant photosensible à des longueurs d'ondes plus longues que celle déclenchant le photoinitiateur selon l'invention, on obtient une réticulation en deux étapes contrôlables. Des amorceurs radicalaires peuvent être par exemple Irgacure® 651 permettant d'amorcer des polymérisations radicalaires à des longueurs d'onde de 365 nm.

L'invention a également pour objet l'utilisation des composés ioniques de l'invention pour les réactions d'amplification chimique de photoresists pour la microlithographie. Lors d'une telle utilisation, un film d'un matériau comprenant un polymère et un composé ionique de l'invention est soumis à une irradiation. L'irradiation provoque la formation de l'acide par remplacement du cation M par un proton, qui catalyse la décomposition ou la transformation du polymère. Après décomposition ou transformation du polymère sur les parties du film qui ont été irradiées, les monomères formés ou le polymère transformé sont éliminés et il reste une image des parties non exposées. Pour cette application particulière, il est avantageux d'utiliser un composé de l'invention qui se présente sous la forme d'un polymère constitué essentiellement d'unités récurrentes styrényles portant un groupement anionique pentacyclique ou dérivé de tétrazapentalène. Ces composés permettent d'obtenir après photolyse des produits qui ne sont pas volatils, et donc pas odorants lorsqu'il s'agit de sulfures. Parmi les polymères qui peuvent ainsi être modifiés en présence d'un composé de l'invention, on peut citer notamment les polymères contenant des motifs ester ou des motifs aryléther de tertioalkyle, par exemple les poly(phtalaldéhydes), les polymères de bisphénol A et d'un diacide, le polytertiobutoxycarbonyl oxystyrène, le polytertiobutoxy-α-méthyl styrène, le polyditertiobutylfumarate-co-allyltriméthylsilane et les polyacrylates d'un alcool tertiaire, en particulier le polyacrylate de tertiobutyle. D'autres polymères sont décrits dans J.V. Crivello et al, Chemistry of Materials 8, 376-381, (1996).

Les composés ioniques de la présente invention, qui présentent une grande stabilité thermique, offrent de nombreux avantages par rapport aux sels connus de l'art antérieur. Ils ont notamment des vitesses d'amorçage et de propagation comparables ou supérieures à celles obtenues à l'aide des anions de coordination de type PF₆⁻, AsF₆⁻ et surtout SbF₆⁻.

Dans les composés de la présente invention, les paires d'ions présentent un très forte dissociation, ce qui permet l'expression des propriétés catalytiques intrinsèques du cation M^{m+}, dont les orbitales actives sont facilement exposées aux substrats de la réaction, ceci dans des milieux variés. La plupart des réactions importantes de la chimie organique peuvent être ainsi effectuées dans des conditions peu contraignantes, avec d'excellents rendements et la facilité de séparer le catalyseur du milieu réactionnel. La mise en évidence d'induction asymétrique par l'utilisation d'un composé ionique selon l'invention qui portent un groupement chiral est particulièrement importante de part sa généralité et sa facilité de mise en oeuvre.

La présente invention a par conséquent comme autre objet l'utilisation des composés de l'invention comme catalyseurs dans les réactions de Friedel et Craft, les réactions de Diels et Alder, les réactions d'aldolisation, les additions de Michael, les réactions d'allylation, les réactions de couplage pinacolique, les réactions de glycosilation, les réactions d'ouvertures de cycles des oxétanes, les réactions de méthathèse des alcènes, les polymérisations de type Ziegler-Natta, les polymérisations du type méthathèse par ouverture de cycle et les polymérisations du type méthathèse de diènes acycliques. Les composés ioniques de l'invention préférés pour une utilisation en tant que catalyseur pour les réactions ci-dessus sont ceux dans lesquels le cation est choisi parmi le lithium, le magnésium, le cuivre, le zinc, l'étain, les métaux trivalents, y compris les terres rares, les platinoïdes, et leurs couples organométalliques, en particulier les métallocènes.

Les composés de l'invention peuvent également être utilisés comme solvant pour effectuer des réactions chimiques, photochimiques, électrochimiques, photoélectrochimiques. Pour cette utilisation particulière, on préfère les composés ioniques dans lesquels le cation est un imidazolium, un triazolium, un pyridinium ou un 4-diméthylamino-pyridinium, ledit cation portant éventuellement un substituant sur les atomes de carbone du cycle. Les composés étant utilisés sous leur forme liquide, on préfère tout spécialement ceux qui ont un point de fusion inférieur à 150°C, plus particulièrement inférieur à 100°C.

Les inventeurs ont également trouvé que la charge anionique portée par le groupement pentacyclique ou dérivé de tétrazapentalène exerçait un effet stabilisant sur les conducteurs électroniques de type polymères conjugués, et que l'utilisation d'un composé dans lequel l'un des substituants Y, Y_{C} ou Q_{S} comprenait une longue chaîne alkyle permettait de rendre ces polymères solubles dans les solvants organiques usuels même à l'état dopé. Le greffage de ces charges sur le polymère lui-même donne des polymères dont la charge globale est cationique, solubles dans les solvants organiques et présentant, outre leur stabilité, des propriétés d'anticorrosion vis-à-vis des métaux, l'aluminium et les métaux ferreux. La présente invention a aussi pour objet des matériaux à conduction électronique comprenant un composé ionique de la présente invention dans lequel la partie cationique est un polycation constitué par un polymère conjugué dopé "p". Les composés ioniques préférés pour cette application sont ceux dans lesquels l'un des substituants Q ou Q_{S} contient au moins une chaîne alkyle ayant de 6 à 20 atomes de carbone. On peut citer en outre les composés dans lesquels Y ou Y_{C} représente un noyau aromatique portant un radical alkyle.

Les colorants de type cationique (cyanines) sont utilisés de plus en plus fréquemment comme sensibilisateurs de films photographiques, pour le stockage optique d'information (disques optiques accessibles en écriture), pour les lasers. La tendance de ces molécules conjuguées à s'empiler lorsqu'elles sont en phases solides limite leur utilisation, par suite des variations des propriétés optiques par rapport à la molécule isolée. L'utilisation de composés ioniques de l'invention pour la fabrication de colorants cationiques dont les contre ions, éventuellement fixés à cette même molécule, correspondent aux fonctionnalités de l'invention, permet de réduire les phénomènes d'agrégation, y compris dans des matrices solides polymères et de stabiliser ces colorants. La présente invention a pour autre objet une composition de colorant cationique, caractérisée en ce qu'elle contient un composé ionique selon l'invention. Les composés ioniques particulièrement préférés pour cette application sont ceux dans lesquels la ou les charges négatives du groupement anionique pentacyclique ou dérivé de tétrazapentalène sont soit fixées à la molécule de colorant, soit elles constituent le contre-ion des charges positives du colorant.

Les composés de la présente invention peuvent être obtenus par des procédés de synthèse classiques connus de l'homme de métier. Parmi ces procédés, certains consistent à construire le cycle, d'autres consistent à modifier des cycles existants.

A titre d'exemple, un composé pentacyclique dans lequel deux groupes Xᵢ sont des groupes -C(CN)= et un groupe Xᵢ est un groupe -S(=O)(CF₃)- peut être obtenu par réaction de diaminomaléonitrile et de triflinate de sodium en présence d'un agent de déshydratation, conformément au premier des schémas réactionnels suivants.

Un composé pentacyclique dans lequel deux groupes Xᵢ sont des groupes -C(CN)= et un groupe Xᵢ est un groupe -P(CF₃)₂- peut être obtenu par réaction du 4,5-dicyano-1,3,2-diazaphospholate avec un agent de trifluorométhylation approprié, conformément au second schéma réactionnel ci-après.

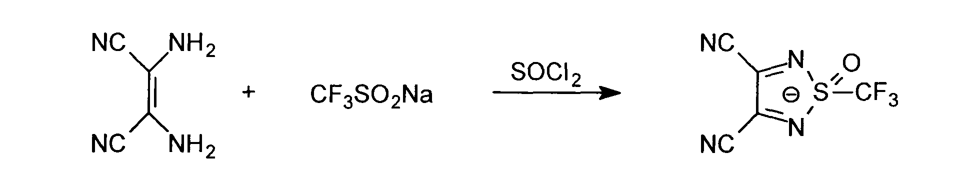

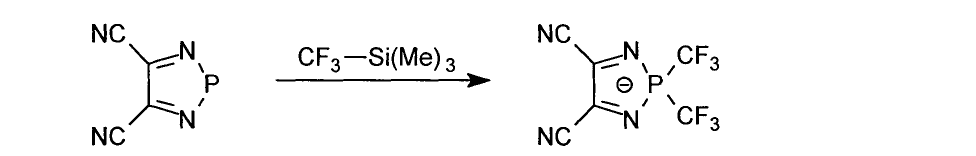

D'autres procédés de préparation sont décrits plus en détail dans les exemples suivants, qui illustrent les composés de l'invention et leurs applications. La présente invention n'est cependant pas limitée à ces exemples particuliers.

### Exemple 1

A 136,11 g (1 mole) de bicarbonate d'aminoguanidine H₂NNHC(=NH)NH₂•H₂CO₃ dans 500 ml de toluène sous agitation, on a ajouté 119,73 g (1,05 moles) d'acide trifluoroacétique CF₃CO₂H. Après addition de l'acide et lorsque le dégagement de CO₂ a cessé, on a effectué une distillation azéotropique à l'aide d'un Dean-Stark. Après 24 heures, on a récupéré 54,5 ml (stoechiométrie 55 ml) d'eau dans le réceptacle du Dean-Stark. Après refroidissement de la solution dans le toluène, il est apparu des cristaux blancs qui ont été récupérés par filtration sur un verre fritté de porosité N°3. Après séchage, on a récupéré 139,9 g (92% de rendement) de 2-amino-5-trifluorométhyl-1,3,4-triazole, ayant une pureté déterminée par RMN du proton et du fluor supérieure à 99%.

Le sel de potassium correspondant a été préparé en faisant réagir le 2-amino-5-trifluorométhyl-1,3,4-triazole avec le carbonate de potassium K₂CO₃ dans l'eau (20% en excès). Après évaporation de l'eau et séchage, le produit obtenu a été repris dans l'acétonitrile, puis l'excès de carbonate a été éliminé par filtration. Après évaporation de l'acétonitrile et séchage, on a obtenu quantitativement le sel de potassium du 2-amino-5-trifluorométhyl-1,3,4-triazole.

30,42 g (200 mmoles) du sel de potassium du 2-amino-5-trifluorométhyl-1,3,4-triazole (obtenu selon le procédé décrit dans l'exemple 1) ont été dissous dans 200 ml d'une solution 1 M d'acide chlorhydrique à 0°C. On a alors ajouté par portions, à la solution sous agitation, 13,8 g (200 mmoles) de nitrite de potassium NaNO₂. Un précipité de diazotrifluorométhyltriazole s'est formé rapidement. Après 15 min, on a ajouté 9,8 g (200 mmoles) de cyanure de sodium NaCN, 35,8 g (400 mmoles) de cyanure de cuivre(I) CuCN et 2 ml de dioxane. On a alors observé un dégagement d'azote. Après une nuit, on a ajouté 13,82 g (100 mmoles) de carbonate de potassium permettant de précipiter le carbonate de cuivre. Après filtration, la solution a été évaporée, le résidu séché, puis repris dans 100 ml de formate de méthyle. Après filtration, évaporation et séchage, le résidu a été repris dans 200 ml d'une solution 1 M d'acide chlorhydrique, puis extrait par deux fractions de 50 ml d'éther. Après séchage de la phase organique par du sulfate de magnésium et évaporation de l'éther, le produit obtenu a été sublimé sous vide secondaire à 40°C. On a récupéré après 48 heures sur le doigt froid 9,89 g (61% de rendement) du 2-cyano-5-trifluorométhyl-1,3,4-triazole, ayant une pureté déterminée par RMN du proton et du fluor supérieure à 99%.

Le sel de potassium correspondant a été préparé en traitant le 2-cyano-5-trifluorométhyl-1,3,4-triazole selon un procédé analogue à celui décrit ci-dessus pour l'obtention du sel de potassium du 2-amino-5-trifluorométhyl-1,3,4-triazole.

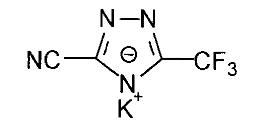

Les sels de sodium et de lithium ont été obtenus par un procédé similaire, en remplaçant le carbonate de potassium respectivement par le carbonate de sodium et le carbonate de lithium.

Ces sels sont solubles dans la plupart des solvants organiques usuels (tétrahydrofurane, acétonitrile, diméthylformamide, acétate d'éthyle, glymes,...) et dans les polymères solvatants aprotiques tels que le poly (oxyde d'éthylène). Dans ce dernier solvant à une concentration O/Li de 14/1, le sel de lithium présente une conductivité ionique supérieure à 10⁻⁴ S.cm⁻¹ à une température de 60°C.

### Exemple 2

Par un procédé analogue à celui décrit dans l'exemple 1, mais en remplaçant l'acide trifluoroacétique par l'acide 5-ène-2,2-difluoroheptanoïque, on a préparé le 2-(4-ène-1,1-difluorobutyl)-5-cyano-1,3,4-triazole avec une pureté, déterminée par RMN du proton et du fluor, supérieure à 99%.

Les sels de potassium, de sodium et de lithium ont été obtenus en traitant le triazole par les carbonates correspondants.

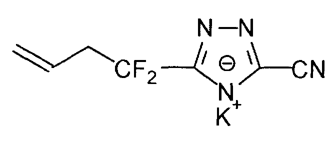

### Exemple 3

Dans 25 ml d'acétonitrile à -20°C, on a dissous 19,02 g (100 mmoles) de sel de potassium du 2-amino-5-trifluorométhyl-1,3,4-triazole préparé selon le procédé de l'exemple 1. Ensuite, on a ajouté par portions 11,68 g (100 mmoles) de tétrafluoroborate de nitrosonium NOBF₄. Après 1 heure sous agitation, le milieu réactionnel a été filtré pour éliminer le précipité de tétrafluoroborate de potassium KBF₄. On a alors ajouté 17,22 g (100 mmoles) de CF₃SO₂K (commercialisé par Parish) en solution dans 25 ml de DMF et une trace de cuivre comme catalyseur. On a observé la formation de fines bulles d'azote dans la solution. Après 48 heures sous agitation, la solution a été évaporée et le résidu recristallisé dans 50 ml d'eau additionnée de 7,46 g (100 mmoles) de chlorure de potassium KCl anhydre. On a obtenu après filtration et séchage, 20,83 g (72% de rendement) du sel de potassium du 2-trifluorométhanesulfonyl-5-trifluorométhyl-1,3,4-triazole, avec une pureté, déterminée par RMN du proton et du fluor supérieure, à 99%.

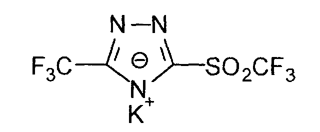

Le sel de lithium a été obtenu par échange ionique dans le THF avec le chlorure de lithium. L'acide a été obtenu par extraction à l'éther d'une solution aqueuse du sel de potassium acidifiée par l'acide chlorhydrique.

On a préparé le sel de scandium en traitant 10 mmoles dudit acide, en solution dans 10 ml d'eau, par 1,67 mmoles d'acétate de scandium. Après une nuit sous agitation, l'eau a été évaporée et on a récupéré quantitativement après séchage le sel de lanthane de ce composé.

### Exemple 4

Dans 100 ml de tétrahydrofurane (THF), on a mis en solution 11,81 g (100 mmoles) de 4,5-dicyanoimidazole et 10,12 g (100 mmoles) de triéthylamine. Après avoir porté la solution à 0°C, on a ajouté lentement, sous argon, 14,06 g de chlorure de benzoyle. Après 6 heures sous agitation, le milieu réactionnel a été filtré pour éliminer le précipité de chlorure de triéthylammonium. On a alors ajouté à la solution 30,21 g (100 mmoles) de fluorure de perfluorobutanesulfonyle C₄F₉SO₂F et 11,22 g (100 mmoles) de 1,4-diazabicyclo[2.2.2]octane (DABCO). Après 72 heures sous agitation, le milieu réactionnel a été filtré pour éliminer le précipité d'hydrochlorure de DABCO, puis le solvant a été évaporé. On a alors repris le résidu dans 100 ml d'une solution 2 M d'hydroxyde de potassium et on a porté la solution au reflux pendant 4 heures. Après refroidissement de la solution, il est apparu un précipité qui a été récupéré par filtration. On a ainsi obtenu 31,56 g (rendement : 72%) du sel de potassium du 2-perfluorobutanesulfonyl-4,5-dicyanoimidazole, avec une pureté, déterminée par RMN du proton et du fluor, supérieure à 99%.

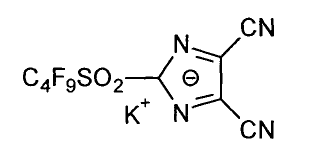

L'acide correspondant a été obtenu par extraction à l'éther d'une solution aqueuse acidifiée du sel de potassium. Le sel de lithium a été obtenu en traitant cet acide par le carbonate de lithium Li₂CO₃.

### Exemple 5

Dans 50 ml d'eau, on a fait réagir 11,81 g (100 mmoles) de 4,5-dicyanoimidazole avec 5,3 g (50 mmoles) de carbonate de sodium Na₂CO₃ anhydre. Après 15 min sous agitation, on a porté la solution à 0°C et on a ajouté 11 g (100 mmoles) du sel de sodium de l'acide dichloroisocyanurique. Après une nuit, la solution a été centrifugée afin d'éliminer le sel de sodium de l'acide isocyanurique qui s'est formé au cours de la réaction. Après addition de 14,91 g (200 mmoles) de chlorure de potassium anhydre, le précipité obtenu a été recristallisé. Après filtration et séchage, on a récupéré 11,88 g (62% de rendement) du sel de potassium du 2-chloro-4,5-dicyanoimidazole ayant une pureté, déterminée par RMN du proton et du fluor supérieure, à 98%.

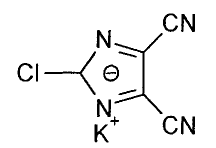

On a obtenu le sel de lithium par échange ionique dans le THF avec le chlorure de lithium.

### Exemple 6

A 8,89 g (40 mmoles)du sel de potassium du 2-(4-ène-1,1-difluoropropane)-5-cyano-1,3,4-triazole, obtenu par un procédé tel que décrit dans l'exemple 2, dans 100 ml d'eau, on a ajouté 6,9 g (40 mmoles) de l'acide 3-chloroperoxybenzoïque, obtenu suivant le procédé décrit par Scwartz & Blumbergs (*J*. *Org*. *Chem*., (1964), 1976). Après 1 heure sous forte agitation, le solvant a été évaporé, puis le résidu a été recristallisé dans 10 ml d'éthanol. Après filtration et séchage, on a récupéré le sel de potassium du 2-(3,4-époxy-1,1-difluorobutane)-5-cyano-1,3,4-triazole, ayant une pureté, caractérisée par RMN du proton et du fluor, supérieure à 98%.

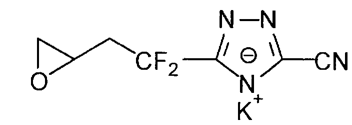

On a obtenu le sel de lithium en traitant le sel de potassium dans le tétrahydrofurane anhydre par la quantité stoechiométrique de chlorure de lithium anhydre, filtration du milieu réactionnel, évaporation du solvant et séchage sous vide.

On a préparé l'homopolymère du sel de potassium du 2-(3,4-époxy-1,1-difluorobutane)-5-cyano-1,3,4-triazole par une polymérisation dans le tétrahydrofurane initiée par voie anionique par le tert-butoxyde de potassium, puis le polysel de lithium par échange ionique dans le THF avec du chlorure de lithium anhydre. Le polysel de lithium présente une conductivité en milieu gélifié (21% en poids de polyacrylonitrile, 38% de carbonate d'éthylène, 33% de carbonate de propylène, 8% de l'homopolymère) de 1,2.10⁻³ S.cm⁻³1 à 30°C. Le nombre de transport cationique dans cette électrolyte est de 0,92. De plus, ce polysel est soluble dans la plupart des solvants organiques usuels (tétrahydrofurane, acétonitrile, diméthylformamide, acétate d'éthyle, glymes,...) et dans les polymères solvatants aprotiques.

### Exemple 7

En boîte à gants sous argon, à une solution dans 100 ml de THF anhydre de 16,02 g (200 mmoles) de succinonitrile NCCH₂CH₂CN et de 41,61 g (200 mmoles) d'hexafluoroacétylacétone CF₃COCH₂COCF₃, on a ajouté par portions 2,38 g d'hydrure de lithium (300 mmoles). Après 48 heures, le milieu réactionnel a été filtré, puis le solvant évaporé. Le résidu a été recristallisé dans 100 ml d'eau additionnée de 14,91 g (200 mmoles) de chlorure de potassium anhydre. Après filtration et séchage, on a obtenu 44,26 g (76% de rendement) du sel de potassium du 2,5-trifluorométhyl-3,4-dicyano-cyclopentadiène, avec une pureté, déterminée par RMN du proton et du fluor, supérieure à 98%.

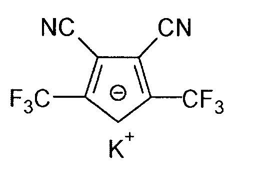

Par un procédé analogue, on a préparé le sel de potassium du :
- 2-*t*-butyl-5-heptafluoropropyl-3,4-dicyano-cyclopentadiène (I) à partir de la 1,1,1,2,2,3,3-heptafluoro-7,7-diméthyl-4,6-octanedione ;
- 2-trifluorométhyl-5-heptafluoropropyl-3,4-dicyano-cyclopentadiène (II) à partir de la 1,1,1,5,5,6,6,7,7,7-decafluoro-2,4-heptanedione ;
- 2-(2-furyl)-5-trifluorométhyl-3,4-dicyano-cyclopentadiène (III), à partir de la 4,4,4-trifluoro-1-(2-furyl)-1,3-butanedione ;
- 2-(2-thiényl)-5-trifluorométhyl-3,4-dicyano-cyclopentadiène (IV), à partir du 1-(2-thénoyl)-3,3,3-trifluoroacétone.

Ces sels peuvent être aisément modifiés en effectuant des réactions de substitution nucléophile sur le carbone ne portant pas de substituant.

On a obtenu les acides par extraction à l'éther de solutions aqueuses des sels de potassium acidifiées par l'acide chlorhydrique.

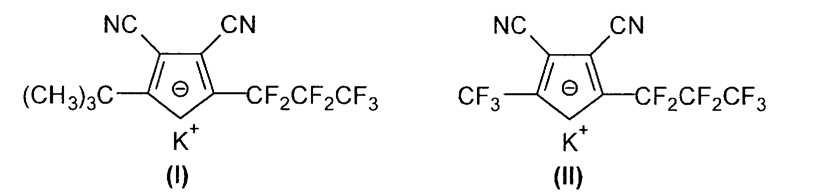

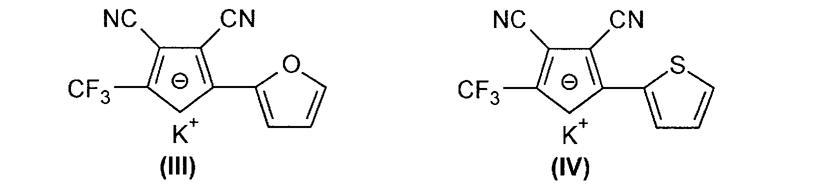

### Exemple 8

Dans 20 ml de THF, on a fait réagir 3,03 g (10 mmoles) de chlorure de l'acide stéarique C₁₇H₃₅COCl et 2, 9 g de 2,5-trifluorométhyl-3,4-dicyano-cyclopentadiène(préparé selon le procédé de l'exemple 7) en présence de 5 ml de pyridine. Après 24 heures sous agitation, la solution a été filtrée pour éliminer le précipité de chlorure de potassium, puis mise en contact avec 500 mg de carbonate de lithium Li₂CO₃. Le mélange a été agité pendant 24 heures, l'excès de carbonate éliminé par centrifugation, puis le solvant évaporé. On a obtenu 5,12 g du sel de lithiulm du 1-stéaryl-2,5-trifluorométhyl-3,4-dicyano-cyclopentadiène ayant une pureté, caractérisée par RMN du proton et du carbone, supérieure à 97%.

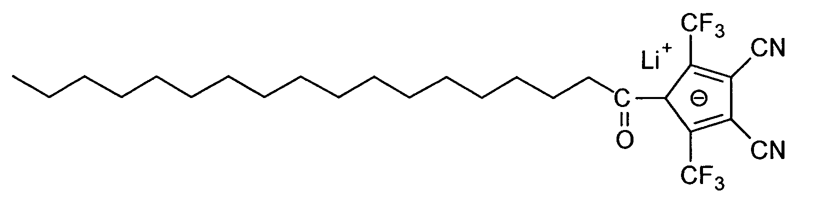

Ce sel possède des propriétés tensio-actives marquées, y compris dans les solvants et polymères solvatants aprotiques.

### Exemple 9

Dans 10 ml de THF, on a fait réagir 324 mg de chlorure de 4-(diméthylamino)azobenzène-4'-sulfonyle (1 mmole) et 290 mg du sel de potassium du 2,5-trifluorométhyl-3,4-dicyano-cyclopentadiène (1 mmole) en présence de 500 µl de triéthylamine. Après 24 heures sous agitation, le précipité de chlorure de potassium a été éliminé et, après évaporation, on a obtenu le sel de triéthylammonium qui a été mis en suspension dans 5 ml d'eau contenant en solution 350 mg de bromure de tétrabutylammonium. Le mélange a été agité pendant 24 heures. On a obtenu une poudre de couleur orange avec une pureté, caractérisée par RMN du proton et du carbone, supérieure à 98%. Cette poudre est soluble dans la plupart des solvants organiques et répond à la formule suivante.

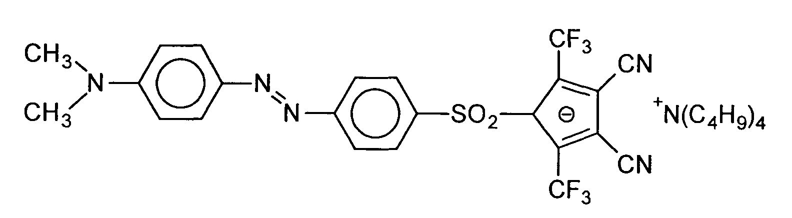

Le composé peut être utilisé comme indicateur de pH en milieu non aqueux (transition jaune-orange - rouge-violet dans la zone de pH 1-4).

### Exemple 10

501 mg (2 mmoles) d'acide 6-hydroxy-2,5,7,8-tétraméthylchroman-2-carboxylique (Trolox®) ont été mis en suspension dans 10 ml d'acétate d'éthyle et 1 ml de pyridine. A ce mélange, on a ajouté 580 mg (2 mmoles) du sel de potassium du 2,5-trifluorométhyl-3,4-dicyano-cyclopentadiène et 313 µl (2 mmoles) de 1,3-diisopropylcarbodiimide. Après 24 heures, le précipité de diisopropylurée a été filtré et le volume de la solution a été réduit à 2 ml à l'aide d'un évaporateur rotatif. On a ajouté 20 ml d'hexane et le mélange a été refroidi à -10°C. Un précipité blanc a été collecté par filtration. Son analyse correspond à C₂₃H₁₇N₂O₃KF₆. Il possède des propriétés anti-oxidantes, en particulier pour les polymères.

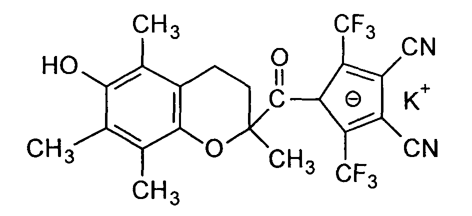

Il en est de même pour les dérivés d'autres cations, y compris de cations organiques tels que les tétraalkylammoniums.

### Exemple 11

2,8 g (10 mmoles) d'acide 4,4'-azobis(4-cyanovalérique) ont été mis en suspension dans 20 ml de formiate de méthyle et 5 ml de pyridine. On a ajouté 5,16 g (20 mmoles) du sel de lithium du 2,5-trifluorométhyl-3,4-dicyano-cyclopentadiène et 4,16 g (20 mmoles) de dicyclohexylcarbodiimide. Le mélange a été maintenu sous agitation magnétique à 0°C pendant 48 heures. Le précipité de dicyclohexylurée a été éliminé par centrifugation et la solution a été évaporée à température ordinaire. On a obtenu un solide cristallin qui est soluble en particulier dans l'acétone, l'acétonitrile, l'acétate d'éthyle, le tétrahydrofurane. Ce composé peut être utilisé comme initiateur radicalaire pour amorcer des réactions de polymérisation ou de réticulation dès 60°C.

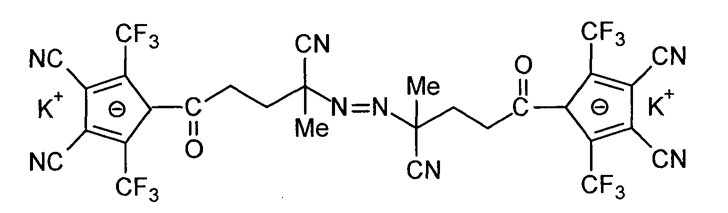

### Exemple 12

Dans un réacteur chimique Parr, on a introduit 200 ml d'acétonitrile anhydre et 13 g (200 mmoles) d'azoture de sodium NaN₃. Après fermeture, le réacteur a été purgé avec de l'azote, puis on a introduit 25 g (154 mmoles, commercialisé par Aldrich) d'hexafluorobutyne CF₃C≡CF₃. Après 24 heures sous agitation, le milieu réactionnel a été filtré et le solvant évaporé. Le résidu a été repris dans 154 ml (154 mmoles) d'une solution 1 M d'acide chlorhydrique, puis extrait par deux fractions de 50 ml d'éther. Après séchage de la phase organique par du sulfate de magnésium et évaporation de l'éther, on a obtenu un produit qu'on a sublimé sous vide à 40°C. On a récupéré après 24 heures sur le doigt froid 27,16 g (86% de rendement) de 4,5-trifluorométhyl-1H-1,2,3-triazole, avec une pureté, déterminée par RMN du proton et du fluor, supérieure à 99%.

On a obtenu le sel de lithium en traitant l'acide par le carbonate de lithium dans l'eau.

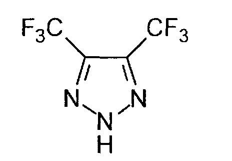

Par un procédé analogue, on a préparé les sels de lithium du :
- 4-trifluorométhyl-5-cyano-1H-1,2,3-triazole (I) à partir du 1-cyano-3,3,3-trifluoropropyne CF₃C≡CCN ;
- 4-pentafluoroéthyl-5-cyano-1H-1,2,3-triazole (II), à partir du 1-cyano-4,4,4,3,3-pentafluorobutyne C₂F₅C≡CCN ;
- 4-heptafluoropropyl-5-cyano-1H-1,2,3-triazole (III), à partir du 1-cyano-5,5,5,4,4,3,3-heptafluoroheptyne C₃F₇C≡CCN.

Les trois alcynes utilisés ont été obtenus suivant la procédure décrite par Huang, Shen, Ding, Zheng (*Tetrahedron Lett.*, (1981), 22, 5283).

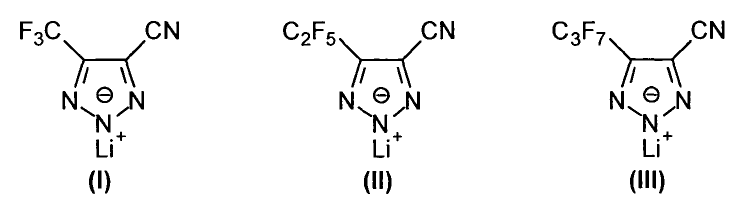

Ces sels sont solubles dans la plupart des solvants organiques usuels (tétrahydrofurane, acétonitrile, diméthylformamide, acétate d'éthyle, glymes,...) et dans les polymères solvatants aprotiques tels que le poly (oxyde d'éthylène). Les solutions concentrées de ces sels dans l'acétone peuvent être utilisées comme catalyseur des réactions de Diels-Alder.

### Exemple 13

Dans 50 ml de DMF, on a fait réagir 13 g (200 mmoles) d'azoture de sodium NaN₃ et 23,29 g (100 mmoles) de 2,3-dichloro-hexafluoro-2-butène CF₃CCl=CClCF₃ en présence de 13,08 g (200 mmoles) de zinc. Après 72 heures sous agitation, le milieu réactionnel a été filtré et le solvant évaporé. Le résidu a été repris dans 154 ml (154 mmoles) d'une solution 1 M d'acide chlorhydrique, puis extrait par deux fractions de 50 ml d'éther. Après séchage de la phase organique par du sulfate de magnésium et évaporation de l'éther, on a obtenu un produit qu'on a sublimé sous vide à 40°C. On a récupéré après 24 heures sur le doigt froid 14,76 g (72% de rendement) de 4,5-trifluorométhyl-1H-1,2,3-triazole, ayant une pureté, déterminée par RMN du proton et du fluor, supérieure à 99%.

Dans 20 ml d'éther, on a dissous 4,1 g de ce composé (20 mmoles), puis 1,38 g de 1,2,3-triazole (20 mmoles). Il s'est immédiatement formé un précipité qui a été récupéré par filtration et séché. On a obtenu le sel suivant :

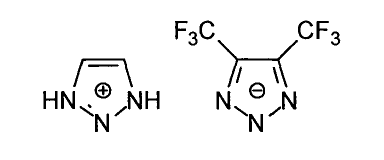

Un mélange molaire de trois 1,2,3-triazole pour un sel de triazolium a été broyé dans un mortier placé dans une boîte à gants. On a obtenu un liquide dans le mortier. Ce sel fondu présente une conductivité protonique élevée supérieure à 10⁻³ S.cm⁻¹ à 30°C. Il peut être utilisé pour préparer un électrolyte polymère, conducteur protonique anhydre, en ajoutant du poly (oxyde d'éthylène), préférentiellement de haute masse ou pouvant être ultérieurement réticulé, au sel fondu sans que cela nuise à la conductivité. Ces électrolytes polymères sont particulièrement intéressants pour la réalisation de systèmes de modulation de la lumière telles que les vitrages électrochromes incluant les systèmes électrochromes à colorants.

Un électrolyte polymère constitué par 80% en poids dudit sel fondu et 20% en poids de polyoxyde d'éthylène de masse 5.10⁶ a été utilisé pour préparer une membrane optiquement transparente dans le visible et ayant une bonne tenue mécanique. On a ensuite réalisé un système électrochrome en boîte à gants en utilisant cette membrane enfermée entre une première électrode constituée par le dépôt sur une plaque de verre d'une couche d'oxyde d'iridium hydrogéné HₓIrO₂ et d'une sous couche conductrice d'oxyde d'étain, et une seconde électrode constituée d'une couche de trioxyde de tungstène WO₃ et d'une sous-couche conductrice d'oxyde d'étain. Cet électrochrome a permis une variation de l'absorption optique entre 80% (état décoloré) et 30% (état coloré) et de bonnes performances en cyclage (plus de 20 000 cycles).

### Exemple 14

Dans 100 ml de THF, on a mis en solution 11,81 g (100 mmoles) de 4,5-dicyanoimidazole et 10,12 g (100 mmoles) de triéthylamine. Après avoir porté la solution à 0°C, on a ajouté lentement, sous argon, 14,06 g de chlorure de benzoyle. Après 6 heures sous agitation, le milieu réactionnel a été filtré pour éliminer le précipité de chlorure de triéthylammonium. On a alors ajouté à la solution 16,85 g (100 mmoles) de chlorure de trifluorométhanesulfonyle et 11,22 g (100 mmoles) de DABCO. Après 72 heures sous agitation, le milieu réactionnel a été filtré pour éliminer le précipité d'hydrochlorure de DABCO, puis le solvant évaporé. On a alors repris le résidu dans 100 ml d'une solution 2 M d'hydroxyde de potassium et on a porté la solution au reflux pendant 4 heures. Après refroidissement de la solution, il est apparu un précipité qui a été récupéré par filtration. On a ainsi obtenu 21 g du sel de potassium du 2-trifluorométhanesulfonyl-4,5-dicyanoimidazole, ayant une pureté, déterminée par RMN du proton et du fluor, supérieure à 99%.

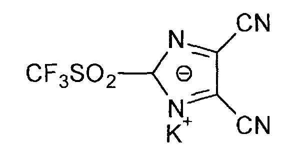

Par un procédé similaire, mais en remplaçant le chlorure de trifluorométhanesulfonyle par le 2,2,2-trifluoroéthyl trifluoroacétate CF₃CO₂CH₂CF₃, on a obtenu le sel de potassium du 2-trifluoroacétyl-4,5-dicyanoimidazole (rendement 69%), avec une pureté, déterminée par RMN du proton et du fluor, supérieure à 99%.

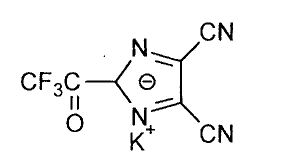

Par un procédé similaire, mais en remplaçant le chlorure de trifluorométhanesulfonyle par le chlorure de trifluoroéthanesulfonyle CF₃CH₂SO₂Cl, on a obtenu le sel de potassium du 2-trifluoroéthanesulfonyl-4,5-dicyanoimidazole (rendement 73%), avec une pureté, déterminée par RMN du proton et du fluor, supérieure à 99%.

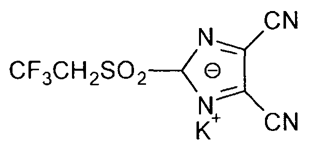

Par un procédé similaire, mais en remplaçant le chlorure de trifluorométhanesulfonyle par le chlorure de sulfamoyle (CH₃)₂NSO₂Cl, on a obtenu le sel de potassium du 2-diméthylaminosulfonyl-4,5-dicyanoimidazole (rendement 73%), avec une pureté, déterminée par RMN du proton et du fluor, supérieure à 99%.

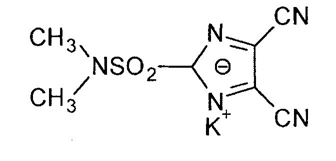

### Exemple 15

En opérant dans une boîte à gants sous argon, à 24,15 g (100 mmoles) de di-2-éthylhexylamine dans 100 ml de THF à -20°C, on a ajouté par portions 32 ml de butyllithium 2M dans le cyclohexane (100 mmoles). Après une heure, on a ajouté 11,85 g (100 mmoles) de fluorure de chlorosulfonyle FSO₂Cl. La réaction s'est poursuivie pendant 4 heures à -20°C, puis pendant 24 heures à température ambiante. On a alors procédé comme dans l'exemple 14 en remplaçant le chlorure de trifluorométhanesulfonyle par le fluorure de di-2-éthylhexylaminosulfonyle en solution dans le THF. On a ainsi obtenu le composé suivant :

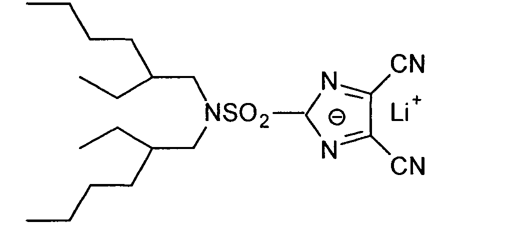

Le sel de potassium a été obtenu en traitant le sel de lithium dans le minimum d'eau par le fluorure de potassium KF. Après filtration, évaporation et séchage, on a récupéré quantitativement le sel de potassium.

Ces sels sont solubles dans la plupart des solvants organiques usuels (tétrahydrofurane, acétonitrile, diméthylformamide, acétate d'éthyle, glymes,...) et dans les polymères solvatants aprotiques.

### Exemple 16

A 13,83 g (100 mmoles) de 1-décyne C₈H₁₇C≡CH, (commercialisé par Aldrich) dans 100 ml de tétrahydrofurane anhydre à -20°C, on a ajouté sous argon durant 30 min, 33,4 ml d'une solution 3 M de chlorure de méthylmagnésium (100 mmoles). Après une heure à -20°C, on a ajouté lentement 30,21 g (100 mmoles) de fluorure de perfluorobutanesulfonyle, puis, après 2 heures à -20°C, on a ajouté par petites portions sur une période d'une heure, 6,5 g (100 mmoles) d'azoture de sodium NaN₃. La réaction s'est poursuivie pendant 3 heures à -20°C, puis pendant 24 heures à température ambiante. Le milieu réactionnel a alors été agité pendant 24 heures avec 4,24 g (100 mmoles)de chlorure de lithium anhydre LiCl. Après centrifugation et filtration du milieu réactionnel sur un verre fritté de porosité N°5, on a récupéré après séchage sous vide 45,52 g (97% de rendement) du sel de lithium du 3-décyne-4-trifluorométhanesulfonyl-1,2,3-triazole avec une pureté, caractérisée par RMN du proton et du fluor, > 96%.

La microanalyse a donné: H 3,77 (3,65) ; Li 1,46 (1,48) ; C 36,45 (35,83) ; N 8,85 (8,95) ; F 35,99 (36,43) ; S 6,75 (6,83).

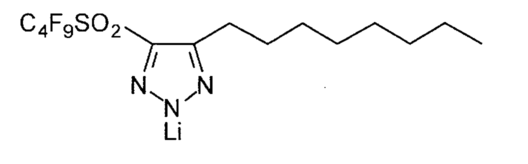

Ce sel est utile comme surfactant. Par exemple, à une concentration aussi faible que 0,1 g/l dans l'eau, la tension superficielle est abaissée à une valeur inférieure à 25 mN/m.

### Exemple 17

En boîte à gants sous argon, à une solution dans 20 ml de THF anhydre de 1,6 g (20 mmoles) de succinonitrile NCCH₂CH₂CN et de 4,52 g (20 mmoles) de 1,1,1,3,5,5,5-heptafluoropentane-2,4-dione CF₃COCH(F)COCF₃, on a ajouté par portions 238 mg (30 mmoles) d'hydrure de lithium. Après 48 heures, le milieu réactionnel a été filtré, puis le solvant évaporé. Le résidu a été recristallisé dans 10 ml d'eau additionnée de 1,49 g (200 mmoles) de chlorure de potassium anhydre. Après filtration et séchage, on a obtenu 4,38 g (71% de rendement) du sel de potassium du 1-fluoro-2,5-trifluorométhyl-3,4-dicyano-cyclopentadiène, ayant une pureté, déterminée par RMN du proton et du fluor, supérieure à 98%.

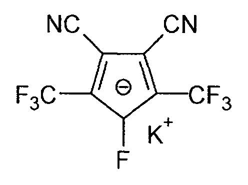

### Exemple 18

On a porté au reflux durant 4 heures, une solution dans 500 ml d'éthanol de 74,08 g (1 mole) d'acétylhydrazide CH₃CONH₂ et de 87,12 g (1 mole) d'acétamidate d'éthyle CH₃C(=NH)OC₂H₅. Après évaporation du solvant, l'acylamidrazone obtenue CH₃C(=NNHCOCH₃)NH₂ a été fondue sous vide à 110°C. Après 24 heures, le produit obtenu a été recristallisé dans le benzène. On a récupéré après filtration et séchage, 59,24 g (61% de rendement) de 3,5-diméthyl-1*H*-1,2,4-triazole, ayant une pureté, déterminée par RMN du proton et du carbone, supérieure à 99%.

On a alors chloré 48,56 g (500 mmoles) de ce composé, mis en solution dans 400 ml de tétrachlorocarbone CCl₄, en faisant passer un courant Cl₂ dans la solution. Après 24 heures, le solvant a été évaporé, le produit séché, puis enfermé dans un réacteur chimique conçu pour effectuer des réactions chimiques dans le fluorure d'hydrogène anhydre HF. Après avoir purgé le réacteur par de l'argon, on a introduit 500 g de fluorure d'hydrogène anhydre (commercialisé par Spolchemie, République Tchèque). Après 72 heures sous agitation, le fluorure d'hydrogène a été évaporé et le produit récupéré dans le réacteur a été sublimé sous vide à 40°C. On a alors obtenu 84 g (82% de rendement) de 3,5-trifluorométhyl-1*H*-1,2,4-triazole, ayant une pureté, caractérisée par RMN du proton et du fluor, supérieure à 99%.

On a préparé le sel de scandium en traitant 10 mmoles de ce composé, en solution dans 10 ml d'eau, par 1,67 mmoles d'acétate de scandium. Après une nuit sous agitation, l'eau a été évaporée et on a récupéré quantitativement le sel de scandium après séchage.

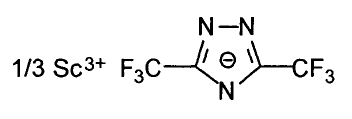

### Exemple 19

On a dégazé par un balayage d'argon sec, une solution contenant 16,96 g (40 mmoles) du sel de lithium du 1-(4-styrènesulfonyl)-2,5-trifluorométhyl-3,4-dicyanoimidazolecyclopentadiène préparé comme à l'exemple 8 en remplaçant le chlorure de l'acide stéarique par le chlorure de 4-styrènesulfonyle (commercialisé par Monomers & Polymers Dajac Laboratories), 3,18 g d'acrylonitrile (60 mmoles) et 100 mg de 1,1'-azobis(cyclohexanecarbonitrile) dans 100 ml de THF anhydre. On a alors chauffé le milieu réactionnel à 60°C pendant 48 heures sous argon pour copolymériser l'acrylonitrile avec le dérivé du styrène. Après refroidissement, la solution a été concentrée, puis le polymère récupéré par reprécipitation dans l'éther. Après filtration et séchage, on a obtenu le polymère suivant :

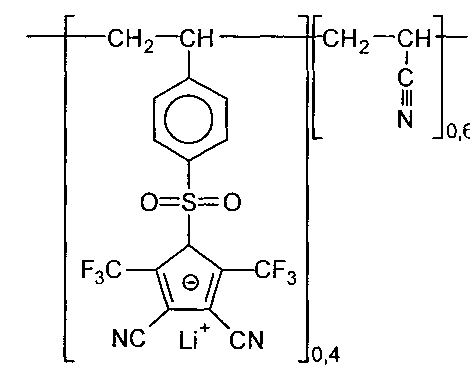

Ce polymère est utile pour les électrolytes polymères gélifiés à anions fixes. Il constitue une matrice sous forme de gel et il se comporte comme un polyélectrolyte.

On a assemblé un générateur électrochimique en superposant les couches suivantes :
- un collecteur de courant en acier inoxydable ayant une épaisseur de 2 mm ;
- une anode composite constituée par un coke de carbone (80% en volume) mélangé avec ledit copolymère comme liant (20% en volume) ;
- ledit copolymère gélifié en tant qu'électrolyte ;
- une cathode composite constituée par du noir de carbone (6% en volume), LiCoO₂ (75% en volume) et ledit copolymère gélifié (20% en volume) ;
- un collecteur de courant analogue au collecteur précité.

Ce générateur a permis d'effectuer 1 000 cycles de charge/décharge entre 3 et 4,2 V en conservant une capacité supérieure à 80% de la capacité au premier cycle, lors d'un cyclage à 25°C. Il présente de très bonnes performances lors d'appel de puissance du fait de l'utilisation d'anions fixes. L'utilisation d'anions fixes a également permis d'améliorer l'évolution de la résistance d'interface.

### Exemple 20

Par un procédé similaire à celui utilisé dans l'exemple 19, on a synthétisé un copolymère d'acrylonitrile (97% en moles) et du sel de lithium du 1-(4-styrènesulfonyl)-2,5-trifluorométhyl-3,4-dicyanoimidazole-cyclopentadiène (3% en moles).

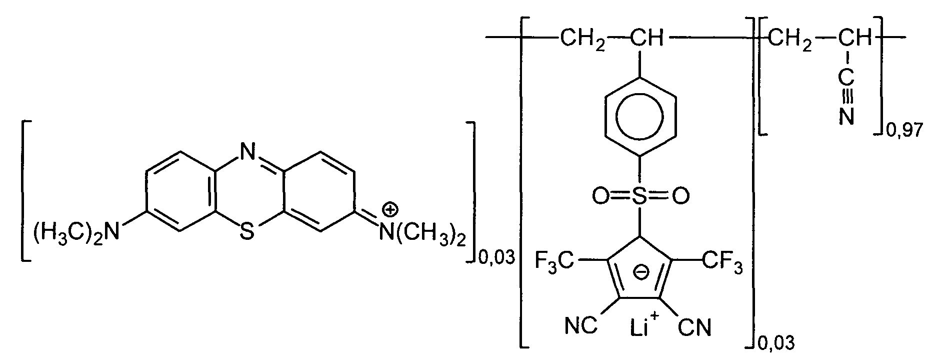

Ce copolymère, sous forme d'un sel de métal alcalin ou d'ammonium, présente des propriétés antistatiques et peut donc avantageusement remplacer les homopolymères d'acrylonitrile qui sont à ce jour largement utilisés sous forme de fibre pour le textile, mais qui ne présentent pas de propriétés antistatiques. De plus, le filage de ce copolymère est plus aisé que celui du PAN non modifié.

Ce copolymère présente de très bonnes interactions avec les colorants cationiques comme le bleu de méthylène, ce qui en fait un matériau d'intérêt pour les fibres textiles colorées. La stabilité de la couleur est nettement améliorée par rapport au copolymère classique d'acrylonitrile et de méthallylsulfonate.

### Exemple 21

A 3,4 g (10 mmoles) de 2-*t*-butyl-5-heptafluoropropyl-3,4-dicyano-cyclopentadiène, obtenu à l'exemple 7, et 821 mg (5 mmoles) de 1,1,3,3-tétraméthoxypropane dans 10 ml d'eau sous agitation, on a ajouté deux gouttes d'acide sulfurique concentré. Après 4 heures sous agitation, on a ajouté 600 mg de carbonate de lithium Li₂CO₃ anhydre, puis après 15 min, 3,22 g (10 mmoles) de bromure de tétrabutylammonium (C₄H₉)₄NBr. Par extraction au dichlorométhane, on a récupéré le composé suivant :

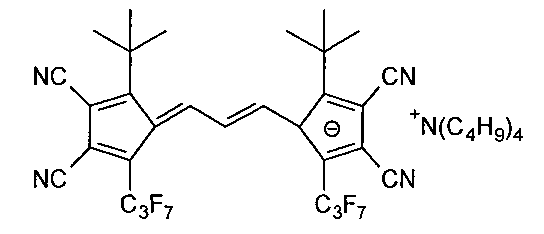

Ce colorant anionique, de la famille des cyanines, absorbant dans le visible, est soluble dans les solvants peu polaires comme le dichlorométhane ou le chlorure de méthylène ainsi que dans les matrices polymères peu polaires comme le polyméthacrylate de méthyle. Le peu d'agrégation des molécules de ce colorant anionique entre elles, évite le phénomène d'élargissement des bandes d'absorption optiques de ce colorant.

### Exemple 22

Dans un mélange de 30 ml de THF et de 10 ml de pyridine, on a fait réagir 18,13 g (50 mmoles) de polyoxyéthylène-23 lauryl éther (Brij® 30) C₁₂H₂₅(OCH₂CH₂)OH et 3,93 g (25 mmoles, commercialisé par Aldrich) de 1,2,3-triazole-4,5-dicarboxylique en présence de 1,03 g (50 mmoles) de 1,3-dicyclohexylcarbodiimide. Après 48 heures, le milieu réactionnel a été filtré pour éliminer le précipité de dicyclohexylurée, puis agité en présence de 5 g de carbonate de lithium Li₂CO₃. Après 48 heures, le milieu réactionnel a été filtré pour éliminer l'excès de carbonate de lithium et le solvant a été évaporé. On a récupéré 20,5 g du composé suivant :

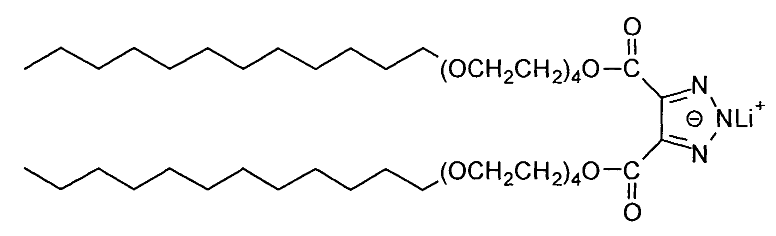

Ce sel est un excellent surfactant. A une concentration aussi faible que 0,1 g/l dans l'eau, la tension superficielle est abaissée à une valeur inférieure à 20 mN/m.

### Exemple 23

Dans 100 ml d'eau, on a mis en suspension 2,54 g du chlorure de polyaniline (AC&T, St Égrève, France):

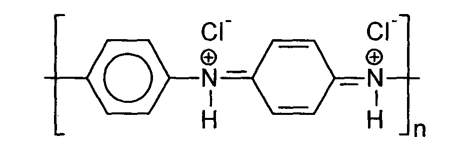

On a alors ajouté 9,51 g du sel de potassium de la trifluorométhanesulfonyl(di-2-éthylhexylaminosulfonyl)imide obtenue à l'exemple 15 :

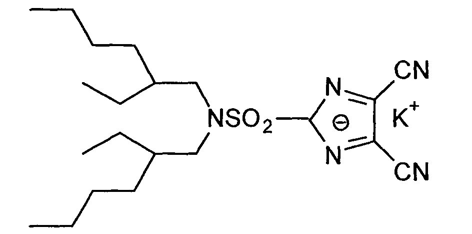

Après 48 heures sous agitation, on a récupéré la polyaniline dopée par le di-2-éthylhexylaminosulfonyl-4,5-dicyanoimidazole. Sous cette forme, elle est soluble dans le toluène et on a pu réaliser un film à partir de cette solution. La polyaniline ainsi dopée est un polymère conducteur électronique qui a une conductivité, mesurée par la méthode des quatres pointes, de 5 S/cm, stable en milieu humide.

On a également réalisé à partir de cette solution un film sur un support de polypropylène (PP) traité par effet Corona. Après séchage sous vide à 60°C pendant 48 heures, on a obtenu un dépôt conducteur et adhérant de polyaniline d'une épaisseur inférieur au micron. Ce type de traitement sur des plastiques est particulièrement intéressant pour la réalisation de contacteurs électriques souples ou de systèmes de protections électromagnétiques. En outre, ce polymère conducteur électronique est un bon inhibiteur de corrosion des métaux ferreux et de l'aluminium en milieu acide ou chlorure.

### Exemple 24

Dans un ballon tricol équipé d'un réfrigérant, d'une agitation mécanique et d'une entrée de gaz neutre (Argon), 9,5 g d'un copolymère de diméthylsiloxane et d'(hydrogéno)-(méthyl)-siloxane (HMS 301 25% SiH, M_{w} 1900 Gelest Inc., Tullytown, PA, USA) ont été mis en solution dans du tétrahydrofurane. On a alors ajouté 7,04 g du sel de lithium du 2-(4-ène-1,1-difluorobutyl)-5-cyano-1,3,4-triazole, préparé comme à l'exemple 2, et 70 mg d'acide chloroplatinique H₂PtCl₆. Le mélange a été chauffé au reflux pendant 4 heures. Le polymère a ensuite été reprécipité dans l'éthanol.

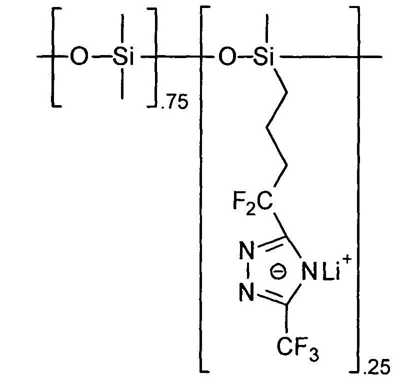

Ce polymère est soluble dans la plupart des solvants organiques, y compris à des teneurs > 2% dans les huiles ou les matériaux siliconés, leur conférant ainsi des propriétés antistatiques.

### Exemple 25

10 mmoles du sel de potassium du 2-perfluorobutanesulfonyl-4,5-dicyanoimidazole, obtenu à l'exemple 4, et 10 mmoles de chlorure de di-4,4'-dodécylphényliodonium (commercialisé par General Electric) ont été agités ensemble durant 24 heures dans l'eau. Par extraction de la phase aqueuse par du dichlorométhane, on a récupéré quantitativement après évaporation du dichlorométhane et séchage, le composé suivant :

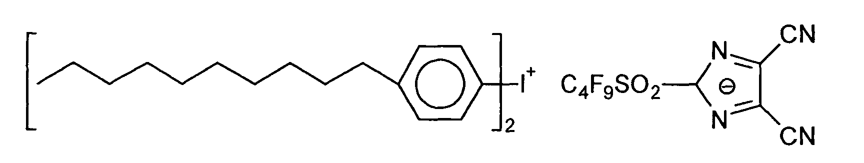

Ce sel permet d'amorcer sous l'effet d'un rayonnement actinique (lumière, rayons γ, faisceaux d'électrons) la réticulation cationique de monomères riches en électrons (éthers vinyliques, alkyl vinyl éthers,...). Il est soluble dans la plupart des solvants organiques usuels (tétrahydrofurane, acétonitrile, diméthylformamide, acétate d'éthyle, glymes,...) et dans les polymères solvatants aprotiques comme le polyoxyde d'éthylène. Il est aussi soluble à plus de 10% en poids dans les solvants réactifs comme le triéthylèneglycol divinyl éther.

On a testé les propriétés de photo-amorçage de ce sel en irradiant par un rayonnement U.V. à 254 nm, d'une puissance de 1900 mW/cm², une solution de triéthylèneglycol divinyl éther contenant à 1% en poids de ce sel. Après quelques secondes sous irradiation, le solvant réactif a pris en masse, cette réaction étant très exothermique.

### Exemple 26

4,08 g (20 mmoles) du sel de potassium du 3, 5-bis (trifluorométhyl)pyrazole (commercialisé par Aldrich) dans 50 ml de THF anhydre ont été traités par 2,17 g (20 mmoles) de 1-chloro-1-éthoxyéthane (préparé suivant la procédure décrite par Grummitt & al., *Organic Synthesis*, Wiley, New-York, 1963, Collect. Vol. IV, p 748). Après 48 heures sous agitation, le milieu réactionnel a été centrifugé pour éliminer le précipité de chlorure de potassium KCl. On a alors ajouté 8,92 g de fluorure de perfluoro(4-méthyl-3,6-dioxaoct-7-ène)-sulfonyle (commercialisé par Apollo Scientific Limited, Stockport, Angleterre) et 4,05 g (40 mmoles) de triéthylamine fraîchement distillée. Le milieu réactionnel alors été porté à 60°C pendant 72 heures, puis le solvant évaporé. Le résidu a été recristallisé dans 30 ml d'eau saturée en chlorure de potassium. Après séchage, on a récupéré le composé suivant :

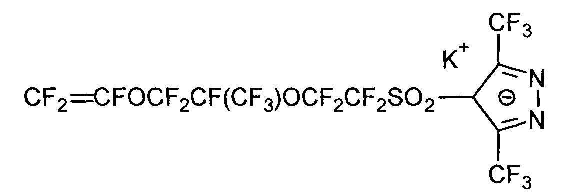

L'acide correspondant a été obtenu par extraction à l'éther d'une solution aqueuse de ce sel de potassium acidifiée par de l'acide chlorhydrique.

On a impregné un textile poreux de GORE-TEX® d'une épaisseur de 100 µm, commercialisé par la société Gore, avec une solution concentrée dans le dichlorométhane dudit acide contenant l'acide cyanovalérique comme initiateur de polymérisation. Après évaporation du solvant, l'acide a été homopolymérisé au sein de la matrice textile en maintenant, sous argon, la température de l'ensemble à 60°C pendant 24 heures. La membrane ainsi obtenue a été utilisée comme électrolyte dans une cellule test de pile à combustible hydrogène/méthanol à électrolyte polymère. On a obtenu une durée de vie de cette membrane supérieure à 1000 heures, avec une plus faible perméabilité au méthanol que celle obtenue en utilisant une membrane de Nafion® 117 (commercialisé par la société Dupont de Nemours) de même épaisseur. Une telle membrane peut aussi être utilisée pour la catalyse hétérogène de Friedel-Crafts de la réaction d'acylation du toluène par le chlorure de benzoyle.

### Exemple 27

A 10 mmoles du sel de lithium du 4-pentafluoroéthyl-5-cyano-1,2,3-triazole, obtenu à l'exemple 12, en solution dans 10 ml d'eau, on a ajouté 12 mmoles de chlorure de 1-éthyl-3-méthyl-1*H*-imidazolium (commercialisé par Aldrich). On a obtenu une phase liquide plus dense que l'eau. Cette phase a été récupérée par extraction au dichlorométhane. Après évaporation du dichlorométhane et séchage sous vide à 40°C du liquide obtenu, on a récupéré le sel liquide suivant :

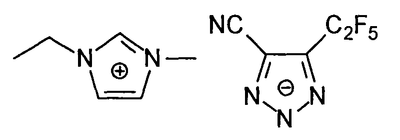

Ce sel fondu présente une conductivité de 4,3.10⁻³ S⁻¹.cm⁻¹ et un point de congélation inférieur à -10°C. Son large domaine de stabilité rédox en fait un électrolyte particulièrement intéressant pour les générateurs électrochimiques tels que les batteries au lithium, les supercapacités, les systèmes de modulation de la lumière, les cellules photovoltaïques.

On a réalisé une cellule photovoltaïque électrochimique similaire dans le principe à celle décrite dans le brevet Européen EP 613466. Pour cela, on a assemblé un système composé de deux électrodes séparées par un espace vide d'une épaisseur de 30 µm. La première électrode a été revêtue d'une couche nanoparticulaire de dioxyde de titane TiO₂ de 0,25 µm d'épaisseur sur laquelle est adsorbé le cis-dithiocyanato-bis-(2,2'-bipyridyl-4,4'-dicarboxylate ruthénium II) comme sensibilisateur. L'espace entre les électrodes a été rempli avec un électrolyte composé du sel fondu dans lequel avait été solubilisé 10% en poids d'iodure de méthylhexyl imidazolium et 10 mmoles d'iode. On a obtenu avec cette cellule photovoltaïque des performances intéressantes et en particulier un courant de court-circuit de 69 µA.cm⁻² et une tension en circuit ouvert de 512 mV.

Ce sel liquide peut également servir d'électrolyte dans les supercapacités électrochimiques utilisant des électrodes de carbone activées ou des électrodes composites obtenues à partir de fibres métalliques et de fibres de carbone traitées en atmosphère réductrice.

### Exemple 28

A 3,2 g (25 mmoles) de 2-(3-thiényl)éthanol dans 60 ml de diméthylformamide anhydre, on a ajouté 7,26 g (25 mmoles) du sel de potassium du 1-vinylsulfonyl-2,5-trifluorométhyl-3,4-dicyano-cyclopentadiène, obtenu par un procédé similaire à celui décrit dans l'exemple 19 en remplaçant le chlorure de 4-styrènesulfonyle par le fluorure d'éthylènesulfonyle (commercialisé par ACROS), 3,46 g de carbonate de potassium K₂CO₃ anhydre (25 mmoles) et 330 mg (1,25 mmoles) d'un éther-couronne, le 18-Crown-6 (agissant comme complexant du cation potassium). Le milieu réactionnel a alors été agité sous argon à 85°C. Après 48 heures, le milieu réactionnel a été filtré sur un verre fritté de porosité N°3, puis le solvant évaporé sous pression réduite. Après séchage, le composé a été recristallisé dans 20 ml d'eau contenant 1,86 g (25 mmoles) de chlorure de potassium KCl anhydre. Après filtration et séchage, on a récupéré le composé suivant :

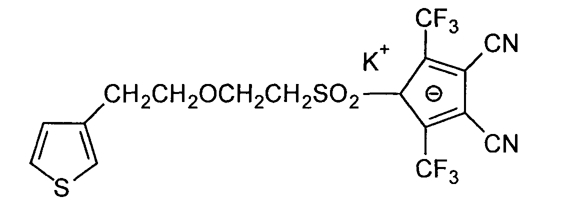

On a préparé 10 ml d'une solution 5.10⁻² M dudit composé dans l'acétonitrile et l'on a effectué une électropolymérisation dans le compartiment anodique d'une cellule électrochimique sur électrode de platine. On a obtenu un film souple conducteur de : 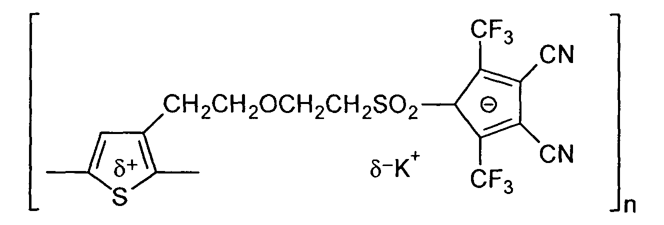 dont le dopage (oxydation) est assuré par échange de cations et d'électrons avec l'extérieur. La conductivité de ce matériau, stable à l'atmosphère ambiante et en milieu humide, est de l'ordre de 10 S.cm⁻¹. L'électropolymérisation effectuée en présence de pyrrole non substitué ou possédant des chaînons oxyéthylène en position N ou 3 donne des copolymères également stables dont le changement de couleur peut être utilisé pour la constitution de systèmes électrochromes.

### Exemple 29

### Catalyse d'une condensation aldolique

L'effet catalytique du sel de scandium du 3-trifluorométhyl-5-trifluorométhanesulfonyl-1,2,4-triazole, obtenu à l'exemple 3, vis à vis d'une condensation aldolique a été évalué de la manière suivante : A une solution contenant 339 mg (0,4 mmoles) du sel de scandium du 3-trifluorométhyl-5-trifluorométhanesulfonyl-1,2,4-triazole (10% en mole) dans 15 ml de dichlorométhane, on a ajouté un mélange de 1,05 mg (6 mmoles) de 1-ène-2-méthyl-1-silylacétal-1-méthoxypropène (CH₃)₂C=C(OSiMe₃)OMe et de 420 mg (4 mmoles) de benzaldéhyde dans 10 ml de dichlorométhane. Après 16 heures sous agitation à l'ambiante, on a ajouté de l'eau et le produit a été extrait avec du dichlorométhane. La phase organique a été lavée par trois fractions de 100 ml d'eau, puis le dichlorométhane évaporé. Le résidu a alors été traité par un mélange tétrahydrofurane/HCl 1 M (20:1) pendant 0,5 heures à 0°C. Après avoir dilué avec de l'hexane, on a ajouté une solution saturée d'hydrogénocarbonate de sodium, puis le produit a été extrait au dichlorométhane. La phase organique a été lavée avec une solution saturée de chlorure de sodium, puis séchée avec du sulfate de sodium. Après évaporation des solvants, le produit brut a été chromatographié sur un gel de silice. On a obtenu le méthyl-3-hydroxy-2,2-diméthyl-phénylpropionate avec un rendement de 90%.

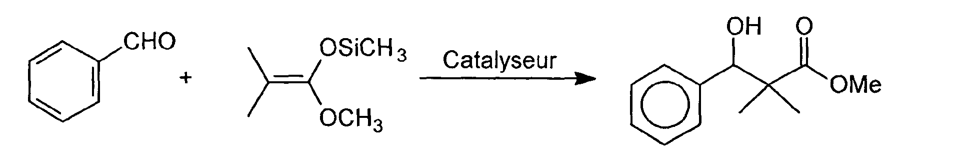

### Exemple 30

### Catalyse d'une addition de Michael

L'effet catalytique du sel scandium 3-trifluorométhyl-5-trifluorométhanesulfonyl-1,2,4-triazole, obtenu à l'exemple 3, vis à vis d'une addition de Michael a été évalué de la manière suivante. A une solution de 339 mg (0,4 mmoles) du sel de scandium du 3-trifluorométhyl-5-trifluorométhanesulfonyl-1,2,4-triazole (10% en mole) dans 15 ml de dichlorométhane, on a ajouté un mélange de 1,05 g (6 mmoles) de 1-ène-2-méthyl-1-silylacétal-1-méthoxypropène (CH₃)₂C=C(OSiMe₃)OMe et de 840 mg (4 mmoles) de chalcone dans 10 ml de dichlorométhane. Après 12 heures sous agitation à l'ambiante, on a ajouté de l'eau et le produit a été extrait avec du dichlorométhane. La phase organique a été lavée par trois fractions de 100 ml d'eau, puis le dichlorométhane a été évaporé. Le résidu a alors été traité par un mélange tétrahydrofurane/HCl 1 M (20:1) pendant 0,5 heure à 0°C. Après avoir dilué avec de l'hexane, on a ajouté une solution saturée d'hydrogénocarbonate de sodium, puis le produit a été extrait au dichlorométhane. La phase organique a été lavée avec une solution saturée de chlorure de sodium, puis séchée avec du sulfate de sodium. Après évaporation des solvants, le produit brut a été chromatographié sur un gel de silice. On a obtenu le composé 1,5-dicarbonylé avec un rendement de 89%.

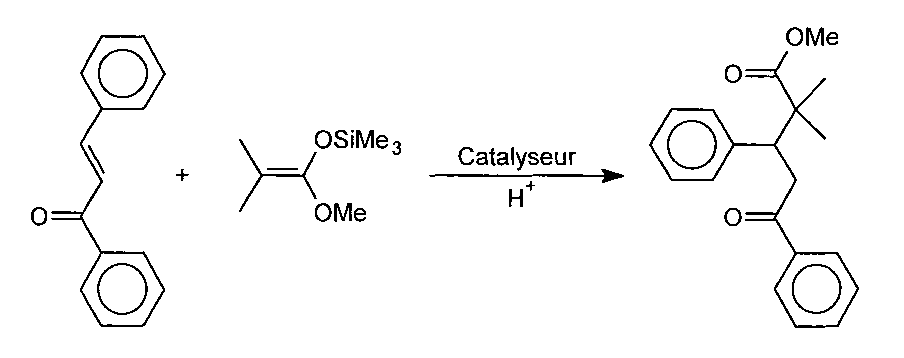

### Exemple 31

### Catalyse d'une réaction d'acylation Friedel-Crafts.

L'effet catalytique du sel de scandium du 3-trifluorométhyl-5-trifluorométhanesulfonyl-1,2,4-triazole, obtenu à l'exemple 3, vis à vis d'une réaction a été évalué de la manière suivante. Dans 40 ml de nitrométhane anhydre, on a ajouté 592 mg (700 µmoles) du sel de scandium du 3-trifluorométhyl-5-trifluorométhanesulfonyl-1,2,4-triazole, puis 1,08 g (10 mmoles) d'anisole et 2,04 g d'anhydride acétique. Après une agitation pendant 10 min à 21°C, le milieu réactionnel a été dilué par 50 ml d'éther et la réaction inhibée par 100 ml d'une solution saturée d'hydrogénocarbonate de sodium NaHCO₃. Après filtration sur de la Celite, la solution a été extraite par trois fractions de 50 ml d'éther, puis la phase éthérée recueillie a été lavée par une solution saturée de chlorure de potassium. Après séchage par du sulfate de magnésium de la phase éthérée et évaporation, on a récupéré 1,46 g de *p*-méthoxyacétophénone (97% de rendement) avec une pureté caractérisée par RMN du proton supérieure à 99%.

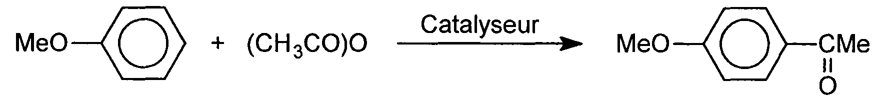

### Exemple 32

Suivant un procédé similaire à celui décrit dans l'exemple 4, on a obtenu le sel de potassium du 2-((1*R*)-(-)-10-camphorsulfonyl-4,5-dicyanoimidazole, en substituant le fluorure de perfluorobutanesulfonyle par le chlorure de (1*R*)-(-)-10-camphorsulfonyle (commercialisé par Aldrich).

Le sel de lithium correspondant a été obtenu par échange ionique (métathèse) dans le tétrahydrofurane avec le chlorure de lithium.

Le sels de scandium a été obtenu en traitant le sel de potassium par une quantité stoechiométrique de tétrafluoroborate de scandium Sc(BF₄)₃ dans l'acétonitrile. Après filtration pour éliminer le précipité de tétrafluoroborate de potassium KBF₄ et évaporation du solvant, on a récupéré quantitativement le composé suivant :

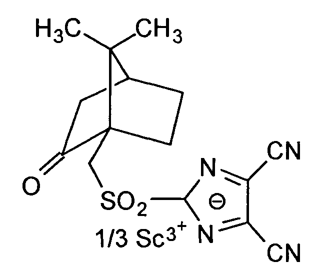

Ce sel a été utilisé en tant que catalyseur d'une réaction de Diels Alder, à savoir une réaction de la méthylvinylcétone avec le cyclopentadiène.

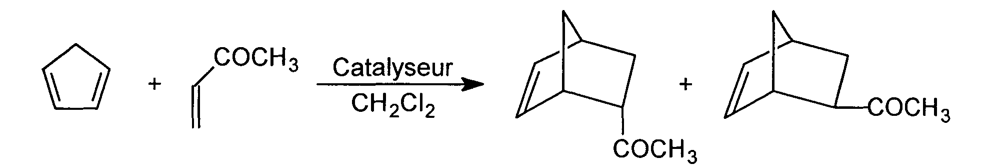

A une solution dans 10 ml de dichlorométhane de 651 mg (10 mmoles) de cyclopentadiène fraîchement distillé et de 701 mg (10 mmoles) de méthylvinylcétone, on a ajouté 200 µmoles du sel de scandium chiral. Après 24 heures à l'ambiante, le milieu réactionnel a été filtré pour éliminer le catalyseur en suspension. Le rendement de la réaction, déterminé par chromatographie en phase gazeuse, est supérieur à 85%. Après une séparation des différents produits de la réaction sur une colonne chirale, on a déterminé par RMN les excès énantiomériques, ce qui a révélé un excès énatiomérique de 73%.

### Exemple 33

Le sel de lithium du 4,5-trifluorométhyl-1,2,3-triazole, obtenu à l'exemple 12 a été testé dans des générateurs électrochimiques de technologie lithium-polymère.

On a réalisé une batterie superposant les couches suivantes :
- un collecteur de courant en acier inoxydable ayant une épaisseur de 2 mm ;
- une cathode constituée par une pastille d'un film de matériau composite ayant une épaisseur de 72 µm et compreanant du dioxyde de vanadium (45% en volume), du noir de Shawinigan (5% en volume) et un polyoxyde d'éthylène de masse M_{w} = 3.10⁵ (50% en volume) ;
- un électrolyte constitué par une pastille d'un film de polyoxyde d'éthylène de masse M_{w} = 5.10⁶ contenant ledit sel de lithium à une concentration O/Li = 15/1 ;
- une anode constituée par une feuille de lithium métallique ayant une épaisseur de 50 µm ;
- un collecteur de courant analogue au collecteur précité.

Les pastilles constituant les électrodes et l'électrolyte ont été découpées en boîte à gants et empilées dans l'ordre indiqué ci-dessus. Les collecteurs ont ensuite été déposés de part et d'autre de l'empilement obtenu.

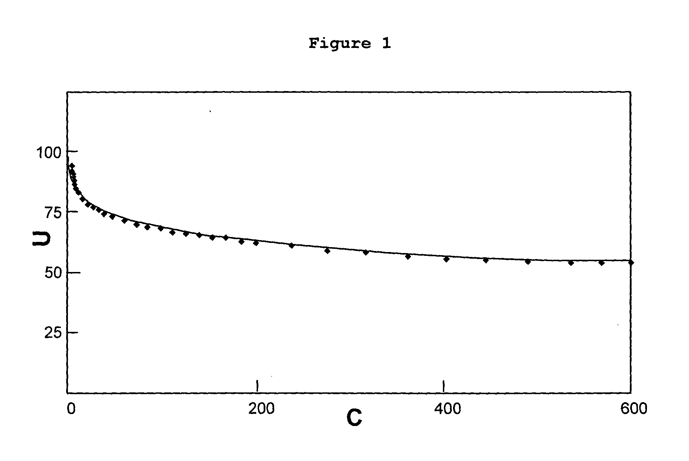

On a scellé le tout dans un boîtier de pile bouton, qui permet à la fois de protéger le générateur de l'atmosphère et d'excercer une contrainte mécanique sur les films. La batterie a alors été placée dans une enceinte sous argon installée dans une étuve à une température de 60°C. Elle a ensuite été cyclée entre 1,8 et 3,3 V à un régime de charge et de décharge de C/10 (capacité nominale chargée ou déchargée en 10 heures). La courbe de cyclage obtenue est donnée sur la figure 1, sur laquelle l'utilisation u, exprimée en %, est donnée en ordonnée, et le nombre de cycles, C, est donné en abscisse.

On a obtenu des performances similaires en utilisant :
- le sel de lithium du 4-trifluorométhyl-5-cyano-1H-1,2,3-triazole obtenu à l'exemple 12 ;
- le sel de lithium du 2-cyano-5-trifluorométhyl-1,3,4-triazole obtenu à l'exemple 1 ;
- le sel de lithium du 2-trifluorométhanesulfonyl-5-trifluorométhyl-1,3,4-triazole obtenu à l'exemple 3 ;
- le sel de lithium du 2-diméthylaminosulphonyl-4,5-dicyanoimidazole obtenu à l'exemple 14 ;
- le sel de lithium du 1-fluoro-2,5-trifluorométhyl-3,4-dicyano-cyclopentadiène obtenu à l'exemple 17 :
   - le polysel de lithium du poly(2-(3,4-époxy-1,1-difluorobutane)-5-cyano-1,3,4-triazole) obtenu à l'exemple 6. Dans ce dernier cas, le polysel est introduit à une concentration O/Li = 25/1 dans l'électrolyte et dans la cathode. On a pu observer de meilleure performances lors d'appel de puissance du fait de l'utilisation d'anions fixés. L'utilisation d'anions fixés a également permis d'améliorer l'évolution de la résistance d'interface.

## Revendications

1. Générateur électrochimique comprenant une électrode négative et une électrode positive séparées par un électrolyte, **caractérisé en ce que** l'électrolyte est un matériau à conduction ionique comprenant un composé ionique en solution dans un solvant, **caractérisé en ce que** le composé ionique comprend au moins une partie anionique associée à au moins une partie cationique M en nombre suffisant pour assurer la neutralité électronique de l'ensemble, **caractérisé en ce que** M est un hydroxonium, un nitrosonium NO⁺, un ammonium -NH₄⁺, un cation métallique ayant la valence m, un cation organique ayant la valence m ou un cation organométallique ayant la valence m, et **en ce que** la partie anionique répond à la formule suivante : 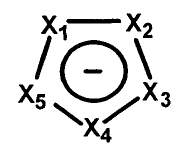 dans laquelle les groupes -Xᵢ- représentent indépendamment les uns des autres un groupe choisi parmi -N=, -N⁻-, -C(Y_{C})=, -C⁻(Y_{C})-, -S(=O)(Q_{S})=, -S(Q_{S})= ou -P(Q') (Q")=, étant entendu que, parmi les cinq groupes -Xᵢ- formant le cycle, au plus quatre groupes -Xᵢ- comprennent un atome d'azote, au plus deux groupes -Xᵢ- comprennent un atome de soufre à condition qu'ils ne soient pas adjacents sur le cycle, au plus un groupe -Xᵢ- comprend un atome de phosphore, et :
- Q' et Q" représentent indépendamment l'un de l'autre un un radical perhaloalkyle ou perhaloalkényle en C₁-C₈, un radical aryle ou alkylaryle en C₆-C₁₂ éventuellement halogéné, chacun pouvant contenir des substitutants oxa, thia, aza ;
- Q_{S} est un radical choisi parmi :
a) les radicaux alkyle ou alkényle, les radicaux aryle, arylalkyle, alkylaryle ou alkénylaryle, les radicaux alicycliques ou hétérocycliques, y compris les radicaux polycycliques, lesdits radicaux étant éventuellement halogénés ou perhalogénés et/ou portant éventuellement au moins un groupe fonctionnel éther, thioéther, amine, imine, amide, carboxyle, carbonyle, isocyanate, isothiocyanate, hydroxy ;
b) les radicaux aromatiques monocycliques, polycyclique ou condensés dans lesquels les noyaux aromatiques et/ou au moins un substituant d'un noyau comprennent des hétéroatomes tels que l'azote, l'oxygène, le soufre ;
c) les radicaux polymères,
d) les radicaux possédant un ou plusieurs groupements ionophores cationiques et/ou un ou plusieurs groupements ionophores anioniques ;
- Y_{C} représente H, ou un groupement attracteur d'électrons choisi dans le groupe constitué par :
* F, Cl, Br, -C≡N, -S-C≡N, -N=C=S, -N=C=O, -NO₂, CₙF₂ₙ₊₁-CₙF₂ₙ₊₁-O-, CₙF₂ₙ₊₁-CH₂-, -OC₂F₄H, -SCF₃, -SCₙF₂ₙ₊₁, -SC₂F₄H, -OCF=CF₂, -SCF=CF₂, FSO₂- ;
* les radicaux QSO₂-, -CO₂Q, Q-N-SO₂-, QCO-, dans lesquels Q est choisi parmi les substituants définis ci-dessus pour Q_{S} ;
* les radicaux comprenant un ou plusieurs noyaux aromatiques contenant éventuellement au moins un atome d'azote, d'oxygène, de soufre ou de phosphore, lesdits noyaux pouvant être éventuellement des noyaux condensés et/ou lesdits noyaux pouvant éventuellement porter au moins un substituant choisi parmi les halogènes, -CN, -NO₂, -SCN, -N₃, CF₂=CF-O-, les radicaux R_{F}- et R_{F}CH₂- dans lesquels R_{F} est un radical perfluoroalkyle ayant de 1 à 12 atomes de carbone, les groupes fluoroalkyloxy, les groupes fluoroalkylthioxy, les radicaux alkyles, alkényles, oxa-alkyles, oxa-alkényles, aza-alkyles, azaalkényles, thia-alkyles, thia-alkényles, les radicaux polymères, les radicaux possédant au moins un groupement ionophore cationique et/ou au moins un groupement ionophore anionique ;
- ou bien deux substituants parmi Y_{C}, Q_{S}, Q' et Q" forment ensemble un cycle ayant de 4 à 8 chaînons, ledit cycle étant éventuellement de nature conjuguée aromatique ;
- ou bien l'un parmi les substituants Y_{C} ou Q_{S} est un radical multivalent (y compris un dendrimère) relié à au moins un deuxième groupement anionique pentacyclique ou dérivé de tétrazapentalène ;
- ou bien l'un des substituants Y_{C} ou Q_{S} représente une unité récurrente d'un polymère.
étant entendu que l'un au moins des substituants Y_{C} ou Q_{S} du composé ionique comprend un dipôle dissociant.

2. Générateur selon la revendication 1, **caractérisé en ce que** le dipôle dissociant est un groupe nitrile.

3. Générateur selon la revendication 1, **caractérisé en ce que** le cation M est un cation métallique choisi dans le groupe constitué par les cations de métaux alcalins, les cations de métaux alcalino-terreux, les cations de métaux de transition, les cations de métaux trivalents, les cations de terres rares et les cations organométalliques.

4. Générateur selon la revendication 3, **caractérisé en ce que** M est un cation métallique choisi dans le groupe constitué par les cations de métaux alcalins.

5. Générateur selon la revendication 3 **caractérisé en ce que** M est un cation Li⁺.

6. Générateur selon la revendication 1, **caractérisé en ce que** Y_{C} comprend un hétérocycle, dérivé de la pyridine, de la pyrazine, de la pyrimidine, de l'oxadiazole ou du thiadiazole, fluoré ou non.

7. Générateur selon la revendication 1, **caractérisé en ce que** l'un au moins des substituants Q_{S} ou Q est choisi parmi les radicaux alkyle, alkényle, oxa-alkyle, oxa-alkényle, azaalkyle, azaalkényle, thiaalkyle ou thiaalkényle ayant de 1 à 24 atomes de carbone, ou parmi les radicaux aryle, arylalkyle, alkylaryle ou alkénylaryle ayant de 5 à 24 atomes de carbone, ou parmi les radicaux alkyles ou alkényles ayant de 1 à 12 atomes de carbone et comprenant éventuellement au moins un hétéroatome O, N ou S dans la chaîne principale ou dans une chaîne latérale, et/ou portant éventuellement un groupe hydroxy, un groupe carbonyle, un groupe amine, un groupe carboxyle.

8. Générateur selon la revendication 1, **caractérisé en ce que** l'un au moins des substituants Q_{S} ou Q est partie d'un radical poly(oxyalkylène) ou d'un radical polystyrène.

9. Générateur selon la revendication 1, **caractérisé en ce que** l'un au moins des substituants Y_{C} ou Q_{S} représente une unité récurrente d'une chaîne polymère.

10. Générateur selon la revendication 1, **caractérisé en ce que** l'un au moins des substituants Y_{C} ou Q_{S} représente un radical ayant une valence v supérieure à deux, comportant à chacune de ses extrémités libres un groupe anionique pentacyclique ou dérivé de tétrazapentalène, ledit radical multivalent comprenant au moins un groupe -SO₂-, un groupe -CO-, un groupe perfluoroalkylène ayant de 2 à 8 atomes de carbone, un groupe phénylène éventuellement substitué par des hétéroatomes, un groupement -(W=W)ₙ- ou un groupement cationique -(W=W)ₙ-W⁺-, dans lesquels W est un atome d'azote ou un groupement CR dans lequel R représente un atome d'hydrogène ou un radical organique et 0≤n≤5, R étant un radical alkyle ayant de 1 à 8 atomes de carbone, ou deux substituants R formant ensemble un cycle.

11. Générateur selon la revendication 1, **caractérisé en ce que** les substituants Y_{C} sont différents d'un groupe perfluoroalkylsulfonyle lorsque le cation est un cation métallique.

12. Générateur selon la revendication 1, **caractérisé en ce que** l'un au moins des substituants Y_{C} ou Q_{S} du composé ionique comprend un groupe alkyle, un groupe aryle, un groupe alkylaryle ou un groupe arylalkyle ; un groupe mésomorphe ou un groupe comprenant au moins une insaturation éthylénique et/ou un groupe condensable et/ou un groupe dissociable par voie thermique, par voie photochimique ou par dissociation ionique ; un polymère conducteur électronique autodopé ; un alcoxysilane hydrolysable ; un piège à radicaux libres ; un dipôle dissociant ; un couple rédox ; un ligand complexant.

13. Générateur selon la revendication 1, **caractérisé en ce que** l'un des substituants Y_{C} du composé ionique est choisi dans le groupe constitué par -OCₙF₂ₙ₊₁, -OC₂F₄H, -SCₙF₂ₙ₊₁ et - SC₂F₄H, -OCF=CF₂, -SCF=CF₂.

14. Générateur selon la revendication 1, **caractérisé en ce que** le solvant est soit un solvant liquide aprotique, choisi parmi les éthers linéaires et les éthers cycliques, les esters, les nitriles, les dérivés nitrés, les amides, les sulfones, les sulfolanes, les sulfamides et les hydrocarbures partiellement halogénés, soit un polymère polaire, soit un de leurs mélanges.

15. Générateur selon la revendication 14, **caractérisé en ce que** le solvant est un polymère solvatant, réticulé ou non, portant ou non des groupements ioniques greffés.

16. Générateur selon la revendication 15, **caractérisé en ce que** le polymère solvatant est choisi parmi les polyéthers de structure linéaire, peigne ou à blocs, formant ou non un réseau, à base de poly(oxyde d'éthylène), les copolymères contenant le motif oxyde d'éthylène ou oxyde de propylène ou allylglycidyléther, les polyphosphazènes, les réseaux réticulés à base de polyéthylène glycol réticulé par des isocyanates, les réseaux obtenus par polycondensation et portant des groupements qui permettent l'incorporation de groupements réticulables et les copolymères à blocs dans lesquels certains blocs portent des fonctions qui ont des propriétés rédox.

17. Générateur selon la revendication 1, **caractérisé en ce que** le solvant est constitué essentiellement par un solvant liquide aprotique et un solvant polymère polaire comprenant des unités contenant au moins un hétéroatome choisi parmi le soufre, l'oxygène, l'azote et le fluor.

18. Générateur selon la revendication 17, **caractérisé en ce que** le polymère polaire contient principalement des unités dérivées de l'acrylonitrile, du fluorure de vinylidène, de la N-vinylpyrrolidone ou du méthacrylate de méthyle.

19. Générateur selon la revendication 1, **caractérisé en ce qu'**il contient en outre au moins un second sel.

20. Générateur selon la revendication 1, **caractérisé en ce qu'**il contient en outre une charge minérale ou organique sous forme de poudre ou de fibres.

21. Générateur selon la revendication 1, **caractérisé en ce que** l'électrode négative est constituée par du lithium métallique, ou par l'un de ses alliages, éventuellement sous forme de dispersion manométrique dans de l'oxyde de lithium, ou par un nitrure double de lithium et d'un métal de transition, ou par un oxyde à bas potentiel ayant pour formule générale Li_{1+y+x/3}Ti_{2-x/3}O₄ (0 ≤ x ≤ 1, 0 ≤ y ≤ 1), ou par le carbone et les produit carbonés issus de la pyrolyse de matières organiques.

22. Générateur selon la revendication 1, **caractérisé en ce que** l'électrode positive est choisie parmi les oxydes de vanadium VOₓ (2 ≤ x ≤ 2,5), LiV₃O₈, Li_{y}Ni₁₋ₓCoₓO₂, (0 ≤ x ≤ 1 ; 0 ≤ y ≤ 1), les spinelles de manganèse Li_{y}Mn₁₋ₓMₓO₂ (M = Cr, Al, V, Ni, 0 ≤ x ≤ 0,5 ; 0 ≤ y ≤ 2), les polydisulfures organiques, FeS, FeS₂, le sulfate de fer Fe₂(SO₄)₃, les phosphates et phosphosilicates de fer et de lithium de structure olivine, ou leurs produits de substitution du fer par le manganèse, utilisés seuls ou en mélanges.

23. Générateur selon la revendication 1, **caractérisé en ce que** le collecteur de la cathode est en aluminium.
